# EUROPÄISCHE PATENTANMELDUNG

(11) **EP 1 972 632 A1**
(43) Veröffentlichungstag der Anmeldung: **24.09.2008**
(21) Anmeldenummer: 07117747.1
(22) Anmeldetag: 02.10.2007
(51) Int. Cl.: C07D 491/08, A23L 1/22

(54) **Oxabicycloalkanone als Riechstoffe**

(30) Priorität: 21.03.2007 EP 07104595
(71) Anmelder: Symrise GmbH & Co. KG, 37603 Holzminden (DE)
(72) Erfinder: Hölscher, Bernd, 37620, Halle (DE)
(74) Vertreter: Eisenführ, Speiser & Partner

(57) **Zusammenfassung**

Die vorliegende Erfindung betrifft Verbindungen der Formel (I) und Formel (Ia) 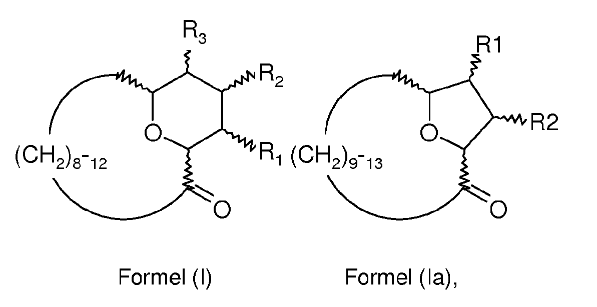 wobei R₁, R₂ und R₃ unabhängig voneinander Wasserstoff oder Methyl bedeuten; Riech- und/oder Aromastoffkompositionen umfassend ein oder mehrere erfindungsgemäße Verbindungen der Formel (I) und/oder Formel (Ia), parfümierte Produkte und aromatisierte Produkte jeweils umfassend ein oder mehrere erfindungsgemäße Verbindungen der Formel (I) und/oder Formel (Ia), sowie Verwendungen einer oder mehrerer erfindungsgemäßer Verbindungen der Formel (I) und/oder Formel (Ia) als Riech- und/oder Aromastoffe sowie Verfahren zu Herstellung von erfindungsgemäßen Verbindungen der Formel (I) und Formel (Ia).

## Beschreibung

Die vorliegende Erfindung betrifft Verbindungen der Formel (I) und Formel (Ia) 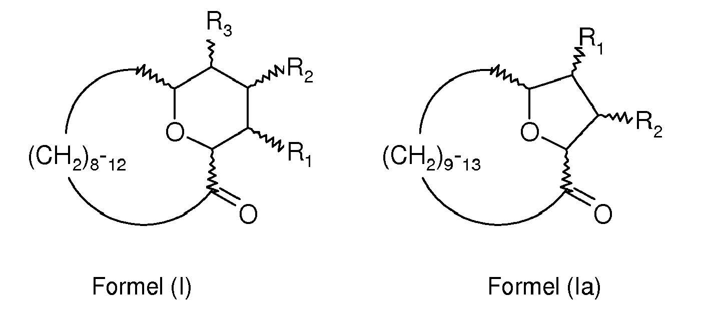 wobei R₁, R₂ und R₃ unabhängig voneinander Wasserstoff oder Methyl bedeuten, Riech- und/oder Aromastoffkompositionen umfassend ein oder mehrere erfindungsgemäße Verbindungen der Formel (I) und/oder Formel (Ia), parfümierte Produkte und aromatisierte Produkte jeweils umfassend ein oder mehrere erfindungsgemäße Verbindungen der Formel (I) und/oder Formel (Ia), sowie Verwendungen einer oder mehrerer erfindungsgemäßer Verbindungen der Formel (I) und/oder Formel (Ia) als Riech- und/oder Aromastoffe sowie Verfahren zu Herstellung von erfindungsgemäßen Verbindungen der Formel (I) und Formel (Ia).

Trotz einer Vielzahl bereits vorhandener Riech- und/oder Aromastoffe besteht in der Parfümindustrie auch weiterhin ein genereller Bedarf an neuen Riech-und/oder Aromastoffen. So besteht ein Bedarf an Riech- und/oder Aromastoffen mit interessanten Duftnoten in Richtung Moschus, die in der Lage sind neben einer moschusartigen Duftnote weitere interessante Geruchsnoten in Riech-und/oder Aromastoffkompositionen zu erzeugen und mit ihren neuartigen bzw. originellen Dufteigenschaften die Möglichkeiten des Parfümeurs zu erweitern. Insbesondere besteht ein Interesse an moschusartigen Riech- und/oder Aromastoffen, welche in der Lage sind, eine harmonische Kombination mit blumig duftenden Riechstoffen einzugehen. Vorzugsweise sollte eine Überlagerung der unterschiedlichen geruchlichen Aspekte und Noten erfolgen, um dadurch einen insgesamt komplexen Geruchseindruck zu erzeugen.

Für die Kreation neuartiger moderner Kompositionen besteht ständiger Bedarf an Riechstoffen mit besonderen geruchlichen Eigenschaften, die geeignet sind, als Grundlage für die Komposition von neuartigen modernen Parfüms mit komplexen Geruchscharakter zu dienen. Bevorzugte gesuchte Riechstoffe sollten neben einer moschusartigen Duftnote weitere Noten und Aspekte aufweisen, die ihnen geruchlichen Charakter und Komplexität verleihen.

Die Suche nach geeigneten Riech- und/oder Aromastoffen, die zur vorliegenden Erfindung führte, wurde durch folgende Sachverhalte erschwert:
- Die Mechanismen der Geruchswahrnehmung sind nicht ausreichend bekannt.
- Die Zusammenhänge zwischen der speziellen Geruchswahrnehmung einerseits und der chemischen Struktur des zugehörigen Riechstoffs andererseits sind nicht hinreichend erforscht.
- Häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riech- und/oder Aromastoffen hängt deshalb stark von der Intuition des Suchenden ab.

Daher bestand die dieser Erfindung zugrunde liegende Aufgabe primär darin, moschusartige Riech- und/oder Aromastoffe zu finden, insbesondere solche, welche gepaart sind mit weiteren interessanten und/oder originellen geruchlichen und/oder geschmacklichen Eigenschaften, wodurch die gesuchten Riech-und/oder Aromastoffe die Herstellung neuartiger und origineller Riech- und/oder Aromastoffkompositionen mit besonderen geruchlichen Noten und Aspekten ermöglichen. Insbesondere sollten Riech- und/oder Aromastoffe mit moschusartigen Noten gefunden werden, welche insbesondere zur Kombination mit Riech-und/oder Aromastoffen geeignet sind, welche eine holzige, eine blumige oder einer holzig - blumige Duftnote aufweisen.

Daneben sollten die diese Hauptaufgabe erfüllenden Riech- und/oder Aromastoffe ferner vorzugsweise über ihre primären, nämlich geruchlichen und/oder geschmacklichen, Eigenschaften, hinaus ein oder mehrere zusätzliche positive Sekundäreigenschaften besitzen, wie z.B. eine höhere Stabilität unter bestimmten Anwendungsbedingungen, einen niedrigen Schwellenwert, eine höhere Ausgiebigkeit, ein besseres Haftungsvermögen, eine hohe eigene Substantivität sowie eine Verbesserung der Substantivität weiterer Riech- und/oder Aromastoffe, eine bemerkenswerte Booster-Wirkung oder ein starkes Blooming, so dass sensorisch bemerkenswerte Effekte oder aber auch bessere dermatologische und toxikologische Eigenschaften gegenüber vergleichbaren Riech- und/oder Aromastoffen erzielt werden können.

Die vorliegende primäre Aufgabe wird gelöst durch die Bereitstellung der Verbindungen der Formel (I) und Formel (Ia) 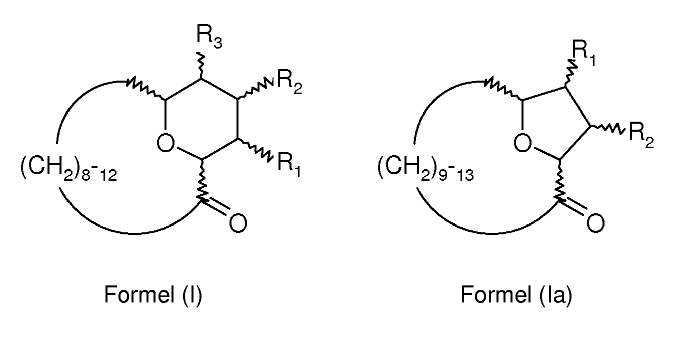 wobei R₁, R₂ und R₃ unabhängig voneinander Wasserstoff oder Methyl bedeuten.

Ein zweiter Gegenstand der vorliegenden Erfindung betrifft eine Riech- und/oder Aromastoffkomposition (Parfümkomposition) umfassend oder bestehend aus
a) einer, zwei, drei, vier oder mehreren erfindungsgemäßen Verbindungen der Formel (I) und/oder Formel (Ia)
   und
b) einem, zwei, drei oder mehreren weiteren Riech- und/oder Aromastoffen, die nicht Verbindungen der Formel (I) und Formel (Ia), wie oben beschrieben darstellen.

Ein dritter Gegenstand der vorliegenden Erfindung betrifft ein parfümiertes Kosmetik- oder Haushalts-Produkt umfassend oder bestehend aus
a) ein, zwei, drei, vier oder mehreren erfindungsgemäßen Verbindungen der Formel (I) und/oder Formel (Ia) und/oder
b) einer, zwei, drei oder mehreren erfindungsgemäßen Riech- und/oder Aromastoffkompositionen
   und
c) ein oder mehreren weiteren Grund-, Hilfs- und/oder Zusatzstoffen.

Ein vierter Gegenstand der vorliegenden Erfindung betrifft aromatisierte Produkte, die der Ernährung, der Mundpflege und/oder dem Genuss dienen sowie Halbfertigware hierzu oder Würzmischungen umfassend oder bestehend aus
a) ein, zwei, drei, vier oder mehreren erfindungsgemäßen Verbindungen der Formel (I) und/oder Formel (Ia), wie oben beschrieben, und/oder
b) einer, zwei, drei oder mehreren erfindungsgemäßen Riech- und/oder Aromastoffkompositionen
   und
c) ein oder mehreren weiteren Grund-, Hilfs- und/oder Zusatzstoffen.

Ein fünfter Gegenstand der vorliegenden Erfindung betrifft die Verwendung einer, zwei, drei, vier oder mehrerer erfindungsgemäßen Verbindungen der Formel (I) und/oder Formel (Ia) als Riech- und/oder Aromastoffe.

Ein sechster Gegenstand der vorliegenden Erfindung betrifft ein Verfahren zum Vermitteln, Modifizieren und/oder Verstärken eines Geruchs oder Geschmacks mit einer, mehreren oder sämtlichen moschusartigen Noten,
wobei eine sensorisch wirksame Menge einer, zwei, drei, vier oder mehrerer erfindungsgemäßen Verbindungen der Formel (I) und/oder Formel (Ia)
oder
eine sensorisch wirksame Menge einer oder mehrerer eine, zwei, drei, vier oder mehrere erfindungsgemäße Verbindungen der Formel (I) und/oder Formel (Ia) umfassende erfindungsgemäße Riechstoff- oder Aromastoffkompositionen mit einem Erzeugnis in Kontakt gebracht oder gemischt wird.

Ein siebter Gegenstand der vorliegenden Erfindung betrifft ein Verfahren zur Herstellung einer oder mehrerer Verbindungen der Formel (I) 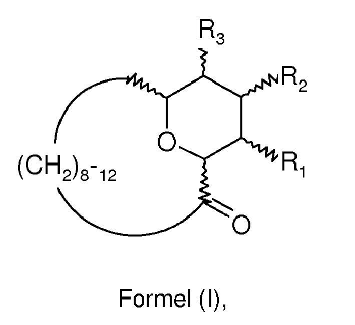 wobei R₁, R₂ und R₃ unabhängig voneinander Wasserstoff oder Methyl bedeuten,
umfassend oder bestehend aus folgenden Schritten:
a) Epoxidation einer Verbindung der Formel (II) zu einer entsprechenden Verbindung der Formel (III) 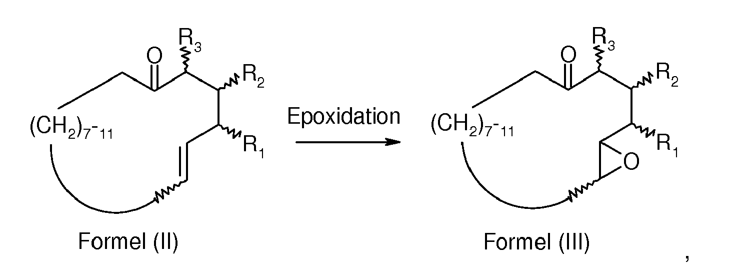 wobei R₁, R₂ und R₃ die oben dargestellten Bedeutungen haben,
b) anschließende Reduktion der entsprechenden Verbindung der Formel (III) zu einer entsprechenden Verbindung der Formel (IV) 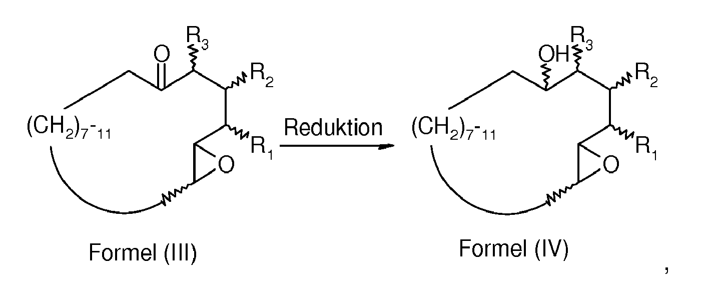 wobei R₁, R₂ und R₃ die oben dargestellten Bedeutungen haben,
c) anschließende Cyclisierung der entsprechenden Verbindung der Formel (IV) zu einer entsprechenden Verbindung der Formel (V) 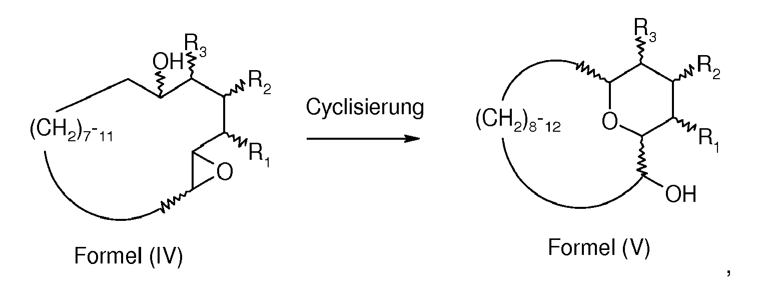 wobei R₁, R₂ und R₃ die oben dargestellten Bedeutungen haben
   und
d) anschließende Oxidation der entsprechenden Verbindung der Formel (V) zu einer entsprechenden Verbindung der Formel (I) 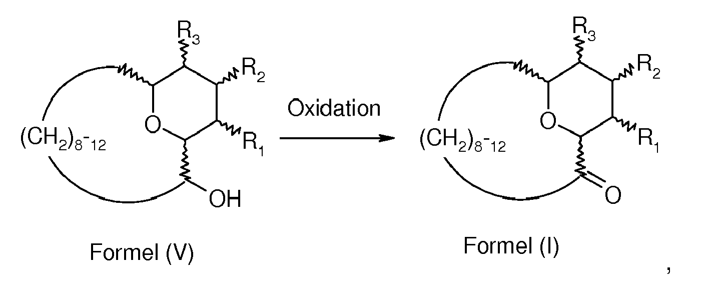
wobei R₁, R₂ und R₃ die oben dargestellten Bedeutungen haben.

Ein achter Gegenstand der vorliegenden Erfindung betrifft ein Verfahren zur Herstellung einer oder mehrerer Verbindungen der Formel (Ia) 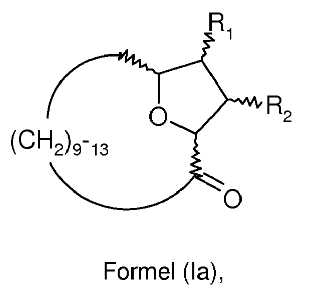 wobei R₁ und R₂ unabhängig voneinander Wasserstoff oder Methyl bedeuten,
umfassend oder bestehend aus folgenden Schritten:
a) Reduktion einer Verbindung der Formel (IIa) zu einer entsprechenden Verbindung der Formel (IIIa) 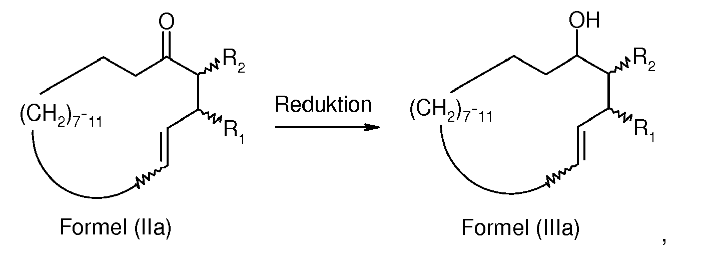 wobei R₁ und R₂ die oben dargestellten Bedeutungen haben,
b) anschließende Epoxidation der entsprechenden Verbindung der Formel (IIIa) zu einer entsprechenden Verbindung der Formel (IVa) 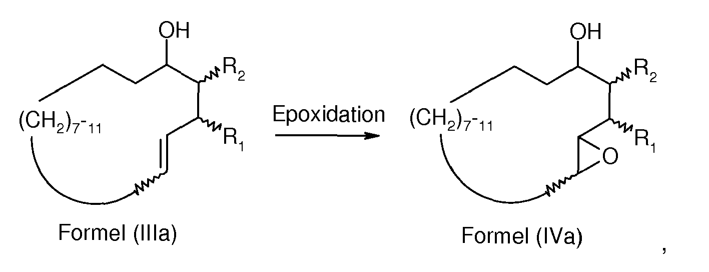 wobei R₁ und R₂ die oben dargestellten Bedeutungen haben,
c) anschließende Cyclisierung der entsprechenden Verbindung der Formel (IVa) zu einer entsprechenden Verbindung der Formel (Va) 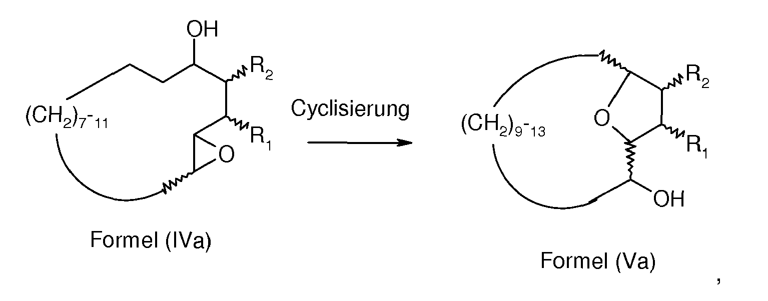 wobei R₁ und R₂ die oben dargestellten Bedeutungen haben
   und
d) anschließende Oxidation der entsprechenden Verbindung der Formel (Va) zu einer entsprechenden Verbindung der Formel (Ia) 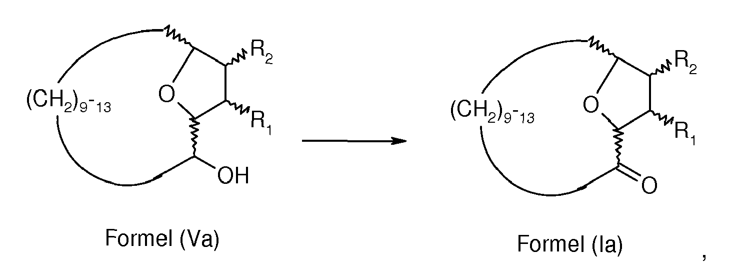
wobei R₁ und R₂ die oben dargestellten Bedeutungen haben.

Weitere Aspekte, insbesondere bevorzugte Aspekte der vorliegenden Erfindung werden in den beigefügten Patentansprüchen sowie der nachfolgenden detaillierten Beschreibung wiedergegeben.

Entsprechend dem Stand der Technik gilt das Gebiet der Riech- und Aromastoffchemie als gut untersucht. Daher ist es besonders überraschend, das auf dem Gebiet der Moschusriechstoffe nunmehr neue, wertvolle Riech- und/oder Aromastoffe gefunden werden konnten. Die erfindungsgemäßen Verbindungen der Formel (I) und Formel (Ia) verfügen über ganz eigenständige olfaktorische Eigenschaften, die sich deutlich von den bekannten Riechstoffen abheben und diese auch übertreffen. Die Eignung der erfindungsgemäß zu verwendenden Verbindungen der Formel (I) und Formel (Ia) als Riech- und/oder Aromastoffe für den Einsatz in der Riech- und Aromenindustrie war bislang nicht bekannt und ist überraschend.

Gemäß vorliegender Erfindung bedeutet der Begriff "Verbindungen der Formel (I) und Formel (Ia)", dass sobald zwei, drei, vier oder mehrere Verbindungen verwendet werden, diese sowohl aus der Gruppe der Formel (I) als auch der Gruppe der Formel (Ia) ausgewählt werden.

Vorzugsweise liegt das Verhältnis der Gesamtmenge von Verbindungen der Formel (I) in einer erfindungsgemäßen Mischung zur Gesamtmenge von Verbindungen der Formel (Ia) im Bereich von 100 : 1 bis 1 : 10 liegt, bevorzugt im Bereich von 3 : 1 bis 1 : 3, besonders bevorzugt im Bereich von 2 : 1 bis 1 : 1.

Gemäß vorliegender Erfindung bedeutet der Begriff "Verbindungen der Formel (I) oder Formel (Ia)", dass ein, zwei, drei, vier oder mehrere Verbindungen ausschließlich aus der Gruppe der Formel (I) oder der Gruppe der Formel (Ia) ausgewählt werden.

Die erfindungsgemäßen Verbindungen der Formel (I) und Formel (Ia) stellen optische Isomere dar und können dementsprechend E- oder Z- konfiguriert sein. Für die vorliegende Erfindung können sowohl die reinen Isomere als auch deren Gemische, insbesondere Racemate eingesetzt werden. Die Geruchseigenschaften können für die E- und Z- Isomere unterschiedlich sein.

Bevorzugt sind für die vorliegendende Erfindung E- oder Z-Isomere sowie deren Racemate der folgenden Verbindungen der Formel (I):
wobei R₁=Methyl sowie R₂ und R₃=Wasserstoff bedeuten oder
R₁ und R₃=Wasserstoff sowie R₂=Methyl bedeuten oder
R₁ und R₂=Wasserstoff sowie R₃=Methyl bedeuten oder
R₁, R₂ und R₃=Wasserstoff bedeuten.

Besonders bevorzugt für die vorliegende Erfindung sind E- oder Z-Isomere sowie deren Racemate der folgenden Verbindungen der Formel (I):
14-Oxa-bicyclo[8.3.1]tetradecan-2-on, 15-Oxa-bicyclo[9.3.1]pentadecan-2-on, 16-Oxa-bicyclo[10.3.1]hexadecan-2-on, 17-Oxa-bicyclo[11.3.1]heptadecan-2-on, 18-Oxa-bicyclo[12.3.1]octadecan-2-on, 13-Methyl-16-Oxa-bicyclo[10.3.1]hexadecan-2-on, 14-Methyl-16-Oxa-bicyclo[10.3.1]hexadecan-2-on und 15-Methyl-16-Oxa-bicyclo[10.3.1]hexadecan-2-on.

Ganz besonders bevorzugt sind:
17-Oxa-bicyclo[11.3.1]heptadecan-2-on und 14-Methyl-16-Oxa-bicyclo[10.3.1] hexadecan-2-on

Von den Isomeren sind gelten die E-Isomere als besonders bevorzugt.

Erfindungsgemäße Verbindungen der Formel (I), insbesondere mit bevorzugten Bedeutungen für R₁, R₂ und R₃, die besonders und die ganz besonders bevorzugten Verbindungen der Formel (I) sowie deren bevorzugte E-Isomere können in allen Gegenständen der vorliegenden Erfindung verwendet werden. Insofern für die Beschreibung der Erfindung, insbesondere im Nachfolgenden, aber auch im Vorstehenden von (erfindungsgemäßen) Verbindungen der Formel (I) gesprochen wird, so fallen hierunter auch alle deren bevorzugte Ausgestaltungen.

Bevorzugt sind für die vorliegendende Erfindung E- oder Z-Isomere sowie deren Racemate der folgenden Verbindungen der Formel (Ia):
wobei R₁=Methyl sowie R₂ =Wasserstoff bedeuten oder
R₁ =Wasserstoff sowie R₂=Methyl bedeuten oder
R₁ und R₂ =Wasserstoff bedeuten.

Besonders bevorzugt für die vorliegende Erfindung sind E- oder Z-Isomere sowie deren Racemate der folgenden Verbindungen der Formel (Ia):
14-Oxa-bicyclo[9.2.1]tetradecan-2-on, 15-Oxa-bicyclo[10.2.1]pentadecan-2-on, 16-Oxa-bicyclo[11.2.1]hexadecan-2-on, 17-Oxa-bicyclo[12.2.1]heptadecan-2-on, 18-Oxa-bicyclo[13.2.1]octadecan-2-on, 13-Methyl-16-Oxa-bicyclo[9.3.1]hexadecan-2-on, 14-Methyl-16-Oxa-bicyclo [11.2.1]hexadecan-2-on und 15-Methyl-16-Oxa-bicyclo[11.2.1]hexadecan-2-on.

Ganz besonders bevorzugt sind:
16-Oxa-bicyclo[11.2.1]hexadecan-2-on und 15-Methyl-16-Oxa-bicyclo[11.2.1] hexadecane-2-on

Erfindungsgemäße Verbindungen der Formel (Ia), insbesondere mit bevorzugten Bedeutungen für R₁ und R₂, die besonders und die ganz besonders bevorzugten Verbindungen der Formel (Ia) können in allen Gegenständen der vorliegenden Erfindung verwendet werden. Insofern für die Beschreibung der Erfindung, insbesondere im Nachfolgenden, aber auch im Vorstehenden von (erfindungsgemäßen) Verbindungen der Formel (Ia) gesprochen wird, so fallen hierunter auch alle deren bevorzugte Ausgestaltungen.

Da die erfindungsgemäßen Verbindungen der Formel (I) und Formel (Ia) neu sind, wurden bislang auch noch keine sensorischen Beschreibung hierzu angegeben. Eigene Untersuchungen haben ergeben, dass erfindungsgemäße Verbindungen der Formel (I) und Formel (Ia) moschusartige Geruchseigenschaften aufweisen.

Die Geruchseigenschaften von 17-Oxa-bicyclo[11.3.1]heptadecan-2-on werden für die einzelnen Isomere wie folgt beschrieben:
Geruchsbeschreibung Z-Isomer: schwacher Moschusgeruch, leicht lederiganimalisch
Geruchsbeschreibung E-Isomer: sehr starker sauberer Moschusgeruch, interessante feine erogene Duftnote, sehr natürlicher Geruchseindruck.

Die Geruchseigenschaften von 14-Methyl-16-Oxa-bicyclo[10.3.1]hexadecan-2-on werden für die einzelnen Isomere wie folgt beschrieben:
Geruchsbeschreibung Isomer 1: angenehmer weicher moschusartiger Geruch
Geruchsbeschreibung Isomer 2: angenehmer weicher moschusartiger Geruch
Geruchsbeschreibung Isomer 3: angenehmer weicher moschusartiger Geruch
Geruchsbeschreibung Isomer 4: angenehmer weicher moschusartiger Geruch.

Die Geruchseigenschaften von 16-Oxa-bicyclo[11.2.1]hexadecan-2-on werden für die einzelnen Isomere wie folgt beschrieben:
Geruchsbeschreibung der E/Z-lsomer: beide haben einen sehr starken sauberen Moschusgeruch mit interessanter feiner erogene Duftnote, sehr natürlicher Geruchseindruck.

Die Geruchseigenschaften von 15-Methyl-16-Oxa-bicyclo[11.2.1]hexadecan-2-on werden für die einzelnen Isomere wie folgt beschrieben:
Geruchsbeschreibung Isomer 1: angenehmer weicher moschusartiger Geruch
Geruchsbeschreibung Isomer 2: angenehmer weicher moschusartiger Geruch
Geruchsbeschreibung Isomer 3: angenehmer weicher moschusartiger Geruch
Geruchsbeschreibung Isomer 4: angenehmer weicher moschusartiger Geruch.

Eine strukturell ähnliche Verbindung, nämlich 9-Oxabicyclo[3.3.1]nonan-2-on, ist aus Eur. J. Org. Chem. 2004, (18), 3884-3892 bekannt. Eine sensorische Beschreibung dieser Verbindung wurde dort allerdings nicht angegeben.

Weitere strukturell ähnliche Verbindungen sind aus der Literatur (A. S. Williams, The Synthesis of Macrocyclic Musks; Synthesis 1999, 1707-1723; G. Ohloff, Riechstoffe und Geruchssinn, Springer Verlag) bekannt. Geruchsbeschreibungen aus eigenen Untersuchungen sowie aus der Literatur lassen sich wie folgt zusammenfassen:
1. 3,4,5,6,7,8,9,10,11,12,13,14-Dodechydro-2H-1-oxa-benzocyclododecen: kein moschusartiger Geruch, schwach holzig
2. Tetradecanhydro-1-oxa-benzocyclododecen: kein moschusartiger Geruch, schwach holzig

Somit lassen diese Geruchsbeschreibungen nicht auf die moschusartigen Geruchs- und/oder Geschmacksnoten der erfindungsgemäßen Verbindungen der Formel (I) und Formel (Ia) schließen.

Gemäß dem zweiten Gegenstand der vorliegenden Erfindung werden ein, zwei, drei, vier oder mehrere erfindungsgemäße Verbindungen der Formel (I) und/oder Formel (Ia) üblicherweise in sensorisch wirksamen Mengen in Riech- und/oder Aromakompositionen eingesetzt, bevorzugt umfasst eine erfindungsgemäße Riech- und/oder Aromastoffkomposition eine Gesamtmenge einer oder mehrerer erfindungsgemäßer Verbindungen der Formel (I) und/oder Formel (Ia) in Komponente (a), die ausreicht in dieser Komposition einen Geruch und/oder Geschmack mit einer, mehreren oder sämtlichen moschusartigen Noten zu vermitteln, zu modifizieren und/oder zu verstärken.

Eine weitere bevorzugte Riech- und/oder Aromastoffkomposition ist dadurch gekennzeichnet, dass die Komposition eine Gesamtmenge einer oder mehrerer erfindungsgemäßer Verbindungen der Formel (I) und/oder Formel (Ia) in Komponente (a) umfasst, die ausreicht
dieser Komposition eine (Aus)Strahlung , Abrundung und/oder Harmonie zu verleihen
und/oder
weitere Geruchs- und/oder Geschmacksnoten dieser Komposition zu verstärken.

Eine weitere bevorzugte erfindungsgemäße Riech- und/oder Aromastoffkomposition ist dadurch gekennzeichnet, dass
ein, zwei, drei oder mehrere Riech- und/oder Aromastoffe der Komponente (b) eine holzige Geruchsnote und/oder Geschmacksnote oder
ein, zwei, drei oder mehrere Riech- und/oder Aromastoffe der Komponente (b) eine blumige Geruchsnote und/oder Geschmacksnote oder
ein, zwei, drei oder mehrere Riech- und/oder Aromastoffe der Komponente (b) eine holzige und blumige Geruchsnote und/oder Geschmacksnote aufweisen.

Erfindungsgemäße Riech- und/oder Aromastoffkompositionen, welche ein, zwei, drei, vier oder mehrere erfindungsgemäße Verbindungen der Formel (I) und/oder Formel (Ia) umfassen oder daraus bestehen, können üblicherweise in flüssiger Form, unverdünnt oder mit einem Lösungsmittel verdünnt zur Parfümierung oder Aromatisierung eingesetzt werden.

Bevorzugt umfassen erfindungsgemäße Riech- und/oder Aromastoffkomposition zusätzlich ein oder mehrere Lösungsmittel. Geeignete Lösungsmittel hierfür sind vorzugsweise Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat und Triacetin.

Eine weitere bevorzugte erfindungsgemäße Riech- und/oder Aromastoffkomposition ist dadurch gekennzeichnet, dass die Komposition teilweise oder vollständig an einen Trägerstoff adsorbiert ist.

Durch diese Adsorption kann bevorzugt eine feine Verteilung der Riech- und/oder Aromastoffe im Produkt und/oder eine kontrollierte Freisetzung bei der Anwendung eingestellt werden. Geeignete Träger werden vorzugsweise ausgewählt aus der Gruppe bestehend aus porösen anorganischen Materialien, wie vorzugsweise Leichtsulfat, Kieselgele, Zeolithe, Gipse, Tone, Tongranulate, Gasbeton usw., oder organischen Materialien, wie vorzugsweise Hölzer, Cellulosebasierende Stoffe, Zucker, Dextrine (besonders bevorzugt Maltodextrin), oder Kunststoffe, wie vorzugsweise PVC, Polyvinylacetate oder Polyurethane. Die Kombination aus erfindungsgemäßer Riech- und/oder Aromastoffkomposition und ein oder mehrere Trägerstoffe stellt ein beispielhaftes erfindungsgemäßen parfümiertes oder aromatisiertes Produkt dar.

Eine weitere bevorzugte erfindungsgemäße Riech- und/oder Aromastoffkomposition ist dadurch gekennzeichnet, dass die Komposition teilweise oder vollständig mikroverkapselt und/oder sprühgetrocknet oder als Einschluss-Komplexe oder als Extrusions-Produkte vorliegt.

Solche Produkte entsprechen entweder einem erfindungsgemäßen parfümierten oder aromatisierten Produkt oder werden dem erfindungsgemäßen parfümierten oder aromatisierten Produkt vorzugsweise in dieser Form hinzugefügt.

Die Mikroverkapselung der erfindungsgemäßen Riech- und/oder Aromastoffkompositionen zu erfindungsgemäßen parfümierten oder aromatisierten Produkten kann vorzugsweise durch das sogenannte Koazervationsverfahren mit Hilfe von Kapselmaterialien, vorzugsweise aus polyurethanartigen Stoffen oder Weichgelatine, erfolgen.

Die sprühgetrockneten erfindungsgemäßen Riech- und/oder Aromastoffkompositionen können vorzugsweise durch Sprühtrocknung einer die Riech- und/oder Aromastoffkomposition enthaltenden Emulsion oder Dispersion hergestellt werden, wobei als Trägerstoffe vorzugsweise modifizierte Stärken, Proteine, Dextrin und pflanzliche Gummen verwendet werden können.

Einschluss-Komplexe können vorzugsweise durch Eintragen von Dispersionen erfindungsgemäßer Riech- und/oder Aromastoffkomposition zusammen mit Cyclodextrinen oder Harnstoffderivaten in ein geeignetes Lösungsmittel, vorzugsweise Wasser, hergestellt werden.

Extrusions-Produkte können durch Verschmelzen erfindungsgemäßer Riech-und/oder Aromastoffkompositionen mit einem geeigneten wachsartigen Stoff und Extrusion mit nachfolgender Erstarrung, ggf. in einem geeigneten Lösungsmittel, vorzugsweise Isopropanol, erhalten werden.

Eine derart modifizierte bevorzugte erfindungsgemäße Riech- und/oder Aromastoffkomposition wird bevorzugt mit geeigneten Materialien zur gezielten Riech-und/oder Aromastofffreisetzung beschichtet.

Durch Beschichtung, sog. "Coaten", mit geeigneten Materialien werden die Eigenschaften der erfindungsgemäßen Riech- und/oder Aromastoffkompositionen im Hinblick auf eine gezielter Duftfreisetzung weiter optimiert, wobei vorzugsweise wachsartige Kunststoffe, wie Polyvinylalkohol, verwendet werden. Die resultierenden Produkte stellen wiederum erfindungsgemäße parfümierte oder aromatisierte Produkte dar.

Bei den weiteren Riech- und/oder Aromastoffen gemäß Komponente (b) der erfindungsgemäßen Riech- und/oder Aromastoffkompositionen handelt es sich üblicherweise um sonstige Riech- und/oder Aromastoffe. Das Gewichtsverhältnis der Gesamtmenge an erfindungsgemäß zu verwendenden Verbindungen der Formel (I) und/oder Formel (Ia) aus Komponente (a) zu der Gesamtmenge an weiteren Riech- und/oder Aromastoffen der Komponente (b) liegt vorzugsweise im Bereich von 1: 1000 bis 1: 0,5.

In erfindungsgemäßen Riech- und/oder Aromastoffkompositionen vermögen ein, zwei, drei, vier oder mehrere einzusetzende erfindungsgemäßen Verbindungen der Formel (I) und/oder Formel (Ia) in Mischung mit weiteren ein, zwei, drei oder mehreren Riech- und/oder Aromastoffen bereits in geringen Dosierungen die Intensität der weiteren Riech- und/oder Aromastoffen zu verstärken und/oder das Gesamtbild der Riechstoffmischung geruchlich abzurunden und/oder zu harmonisieren und/oder der Mischung mehr (Aus)Strahlung sowie Natürlichkeit zu verleihen.

Durch Kombination einer, zwei, drei, vier oder mehrerer erfindungsgemäßer Verbindungen der Formel (I) und/oder Formel (Ia) mit einem, zwei, drei oder mehreren weiteren Riech- und/oder Aromastoffen mit vorzugsweise holzigem und/oder blumigem Geruch und/oder Geschmack lassen sich neue Riech-und/oder Aromastoffkompositionen bilden. Auf diese Weise lassen sich besonders interessante und natürliche neue und originelle Duftnoten kreieren. Bevorzugte weitere (sonstige) Riech- und/oder Aromastoffe der Komponente (b) der erfindungsgemäßen Riech- und/oder Aromastoffkomposition, die zur Kombination mit ein oder mehreren erfindungsgemäßen Verbindungen der Formel (I) und/oder Formel (Ia) vorzugsweise geeignet sind, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001. Im einzelnen seien als besonders bevorzugte weitere Riech- und/oder Aromastoffe der Komponente (b) der erfindungsgemäßen Riech- und/oder Aromastoffkompositionen die Nachfolgenden genannt:
**Extrakte aus natürlichen Rohstoffen,** wie Etherische Öle, Concretes, Absolues, Resine, Resinoide, Balsame, Tinkturen vorzugsweise ausgewählt aus der Gruppe bestehend aus
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-ÖI; Lorbeerblätteröl; Macisöl; Majoranöl; Mandarinenöl; Massoirindenöl; Mimosa-Absolue; Moschuskörneröl; Moschustinktur; Muskateller-Salbei-ÖI; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl (engl.: turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen.
Einzel-Riechstoffe werden vorzugsweise aus der Gruppe ausgewählt bestehend aus
Kohlenwasserstoffe, wie vorzugsweise 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
aliphatische Alkohole, wie vorzugsweise Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
aliphatische Aldehyde und deren Acetale, wie vorzugsweise Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
aliphatische Ketone und deren Oxime, wie vorzugsweise 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
aliphatische schwefelhaltige Verbindungen, wie vorzugsweise 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
aliphatische Nitrile, wie vorzugsweise 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
Ester von aliphatischen Carbonsäuren, wie vorzugsweise (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
acyclische Terpenalkohole, wie vorzugsweise Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
acyclische Terpenaldehyde und -ketone, wie vorzugsweise Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
cyclischen Terpenalkohole, wie vorzugsweise Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
cyclische Terpenaldehyde und -ketone, wie vorzugsweise Menthon; Isomenthon; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton ; 4,6,8-Megastigmatrien-3-on; alpha-Sinensal ; beta-Sinensal ; acetyliertes Cedernholzöl (Methylcedrylketon);
cyclische Alkohole, wie vorzugsweise 4-tert.-Butylcyclohexanol; 3,3,5-Trimethylcyclohexanol; 3-lsocamphylcyclohexanol; 2,6,9-Trimethyl-Z2,Z5,E9-cyclododecatrien-1-ol; 2-Isobutyl-4-methyltetrahydro-2H-pyran-4-ol;
cycloaliphatische Alkohole, wie vorzugsweise alpha,3,3-Trimethylcyclohexylmethanol;l-(4-lsopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
cyclische und cycloaliphatische Ether, wie vorzugsweise Cineol; Cedrylmethylether; Cyclododecylmethylether;l,1-Dimethoxycyclododecan; (Ethoxymethoxy)cyclododecan; alpha-Cedrenepoxid; 3a,6,6,9a-Tetramethyldodecahydronaphtho[2,1 -b]furan; 3a-Ethyl-6,6,9a-trimethyldodecahydronaphtho[2,1 -b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
cyclische und makrocyclische Ketone, wie vorzugsweise 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
cycloaliphatische Aldehyde, wie vorzugsweise 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
cycloaliphatische Ketone, wie vorzugsweise 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
Ester cyclischer Alkohole, wie vorzugsweise 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
Ester cycloaliphatischer Alkohole, wie vorzugsweise 1-Cyclohexylethylcrotonat;
Ester cycloaliphatischer Carbonsäuren, wie vorzugsweise Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
araliphatische Alkohole, wie vorzugsweise Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
Ester araliphatischer Alkohole und aliphatischer Carbonsäuren, wie vorzugsweise Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
araliphatische Ether, wie vorzugsweise 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
aromatische und araliphatische Aldehyde, wie vorzugsweise Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
aromatische und araliphatische Ketone, wie vorzugsweise Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl) ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
aromatische und araliphatische Carbonsäuren und deren Ester, wie vorzugsweise Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
stickstoffhaltige aromatische Verbindungen, wie vorzugsweise 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
Phenole, Phenylether und Phenylester, wie vorzugsweise Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
heterocyclische Verbindungen, wie vorzugsweise 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
Lactone, wie vorzugsweise 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Sofern zwei, drei oder mehrere weitere Riech- und/oder Aromastoffe in Komponente (b) der erfindungsgemäßen Riech- und/oder Aromastoffkompositionen verwendet werden, können diese aus den Extrakten und/oder aus den Einzel-Riechstoffen ausgewählt werden. Werden zwei, drei oder mehrere weitere Riech-und/oder Aromastoffe aus den Einzel-Riechstoffen ausgewählt, können diese alle aus einer oder aus mehreren Gruppen hiervon ausgewählt werden.

Eine erfindungsgemäße Riech- oder Aromastoffkomposition lässt sich beispielsweise herstellen, indem eine, zwei, drei, vier oder mehrere erfindungsgemäße Verbindungen der Formel (I) und/oder Formel (Ia) in Komponente (a) mit einer, zwei, drei oder mehreren weiteren Riech- oder Aromastoffen der Komponente (b) vermischt werden. Die Verbindungen der Komponente (a) werden dabei regelmäßig in einer Menge eingesetzt, die ausreicht, in der fertigen Komposition einen Geruchs- und/oder Geschmacksnote des Typs moschusartig zu vermitteln, zu modifizieren und/oder zu verstärken.

Eine erfindungsgemäße Riech- und/oder Aromastoffkompositionen umfasst vorzugsweise eine Gesamtmenge der erfindungsgemäßen Verbindungen der Formel (I) und/oder Formel (Ia) der Komponente (a) im Bereich von 0,00001 bis 99,9 Gew.-%, vorzugsweise 0,001 bis 70 Gew.-% und besonders bevorzugt 0,01 bis 50 Gew.-%, jeweils bezogen auf die Gesamtmenge der Riech- und/oder Aromastoffkomposition.

Sofern eine, zwei, drei, vier oder mehrere erfindungsgemäße Verbindungen der Formel (I) und/oder Formel (Ia) mit dem Ziel eingesetzt werden, einer erfindungsgemäßen Riech- und/oder Aromastoffkomposition (Aus)Strahlung, Abrundung und/oder Harmonie zu verleihen und/oder bestimmte Geruchs- und/oder Geschmacksnoten weiterer Riech- und/oder Aromastoffe zu verstärken, liegt die Gesamtmenge der Komponente (a) üblicherweise vergleichsweise niedrig und bevorzugt im Bereich von 0,01 bis 5 Gew.-%, besonders bevorzugt im Bereich von 0,1 bis 2 Gew.-%, jeweils bezogen auf die Gesamtmenge der Riech-und/oder Aromastoffkomposition. Sofern innerhalb der bevorzugten Konzentrationsbereiche eine vergleichsweise niedrige Konzentration für ein oder mehrere erfindungsgemäße Verbindungen der Formel (I) und/oder Formel (Ia) gewählt wird, kann es in manchen Fällen in Abhängigkeit von den weiteren Komponenten und deren Gesamtmenge in der jeweiligen Komposition noch nicht zu der Vermittlung der oben angegebenen Eigengeruchs- und/oder -geschmacksnoten der oder einer, zweier oder mehrerer erfindungsgemäßer Verbindungen der Formel (I) und/oder Formel (Ia) kommen.

Insofern für die Beschreibung der Erfindung, insbesondere im Nachfolgenden, aber auch im Vorstehenden von (erfindungsgemäßen) Riech- und/oder Aromastoffkomopistionen gesprochen wird, so fallen hierunter auch alle deren bevorzugte Ausgestaltungen.

In Bezug auf den dritten und vierten Gegenstand der vorliegenden Erfindung, parfümiertes Produkt und aromatisiertes Produkt, werden ein, zwei, drei, vier oder mehrere erfindungsgemäße Verbindungen der Formel (I) und/oder Formel (Ia) und/oder ein, zwei, drei oder mehrere erfindungsgemäße Riech- und/oder Aromastoffkompositionen, insbesondere auch gemäß den bevorzugten Ausgestaltungen, üblicherweise in solchen Mengen eingesetzt, in denen die Gesamtmenge der Verbindungen der Formel (I) und/oder Formel (Ia) sensorisch wirksam ist.

Bevorzugt umfasst ein erfindungsgemäßes parfümiertes oder aromatisiertes Produkt eine Gesamtmenge einer, zwei, drei, vier oder mehrerer erfindungsgemäßer Verbindungen der Formel (I) und/oder Formel (Ia), insbesondere auch in Komponente (a) der erfindungsgemäßen Riech- und/oder Aromastoffkomposition, die ausreicht in dem Produkt einen Geruch und/oder Geschmack mit einer, mehreren oder sämtlichen moschusartigen Noten zu vermitteln, zu modifizieren und/oder zu verstärken.

Ein bevorzugtes parfümiertes oder aromatisiertes Produkt ist dadurch gekennzeichnet, dass das Produkt eine Gesamtmenge einer oder mehrerer erfindungsgemäßer Verbindungen der Formel (I) und/oder Formel (Ia), insbesondere in Komponente (a) einer erfindungsgemäßen Riech- und/oder Aromastoffkomposition umfasst, die ausreicht
diesem Produkt eine (Aus)Strahlung, Abrundung und/oder Harmonie zu verleihen
und/oder

Geruchs- und/oder Geschmacksnoten eines weiteren Riech- und/oder Aromastoffes oder weiterer zwei, drei oder mehrerer Riech- und/oder Aromastoffe des jeweiligen Produktes zu verstärken.

Bevorzugte erfindungsgemäße parfümierte oder aromatisierte Produkte umfassen in Komponente (b) des Produktes erfindungsgemäße Riech- und/oder Aromastoffkompositionen, wie sie insbesondere auch als bevorzugte Ausgestaltungen hierzu oben dargestellt wurden.

Bevorzugte parfümierte Kosmetik- oder Haushaltsprodukte werden ausgewählt aus der Gruppe bestehend aus
Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmitteln vorzugsweise ausgewählt aus der Gruppe bestehend aus Fußbodenreinigern, Fensterglasreinigern, Geschirrspülmittel, Bad- oder Sanitärreiniger, Scheuermilch, festen oder flüssigen WC-Reinigern, pulver- oder schaumförmigen Teppichreinigern, Textilerfrischer, Bügelhilfen, flüssige Waschmitteln, pulverförmige Waschmitteln, Wäschevorbehandlungsmittel, Wäscheweichspüler, Waschseifen und Waschtabletten, Desinfektionsmittel, Oberflächendesinfektionsmittel, Luftverbesserer in flüssiger, gelartiger oder auf einem festen Träger aufgebrachter Form, Aerosolsprays, Wachse und Polituren vorzugsweise ausgewählt aus der Gruppe bestehend aus Möbelpolituren, Fußbodenwachsen und Schuhcremes, Körperpflegemittel vorzugsweise ausgewählt aus der Gruppe bestehend aus festen und flüssigen Seifen, Duschgelen, Shampoos, Rasierseifen, Rasierschäumen, Badeölen und kosmetischen Emulsionen vom Öl-in-Wasser-, vom Wasser-in-Öl- und vom Wasser-in-Öl-in-Wasser-Typ vorzugsweise ausgewählt aus der Gruppe bestehend aus Hautcremes- und -lotionen, Gesichtscremes und -lotionen, Sonnenschutzcremes-und - lotionen, After-sun-cremes und -lotionen, Handcremes und -lotionen, Fußcremes und -lotionen, Enthaarungscremes und -lotionen, After-shave-Cremes und - lotionen, Bräunungscremes und -lotionen, Haarpflegeprodukten vorzugsweise ausgewählt aus der Gruppe bestehend aus Haarsprays, Haargelen, festigenden Haarlotionen, Haarspülungen, permanenten und semipermanenten Haarfärbemittel, Haarverformungsmittel vorzugsweise ausgewählt aus der Gruppe bestehend aus Kaltwellen- und Haarglättungsmittel; Haarwässern, Haarcremes und - lotionen, Deodorantien und Antiperspirantien vorzugsweise ausgewählt aus der Gruppe bestehend aus Achselsprays, Roll-ons, Deosticks und Deocremes, Produkte der dekorativen Kosmetik vorzugsweise ausgewählt aus der Gruppe bestehend aus Lidschatten, Nagellacke, Make-ups, Lippenstifte und Mascara, Kerzen, Lampenöle, Räucherstäbchen, Insektizide, Repellentien und Treibstoffe.

Bevorzugte aromatisierte Produkte, die der Ernährung oder dem Genuss dienend, sowie Halbfertigprodukte hiervon oder Würzmischungen werden vorzugsweise ausgewählt aus der Gruppe bestehend aus Backwaren vorzugsweise ausgewählt aus der Gruppe bestehend aus Brot, Trockenkekse, Kuchen und sonstiges Gebäck, Süßwaren vorzugsweise ausgewählt aus der Gruppe bestehend aus Schokolade, Schokoladenriegelprodukte, sonstige Riegelprodukte, Fruchtgummi, Hart- und Weichkaramellen und Kaugummi, alkoholische oder nicht-alkoholische Getränke vorzugsweise ausgewählt aus der Gruppe bestehend aus Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte und Frucht- oder Gemüsesaftzubereitungen, Instantgetränke vorzugsweise ausgewählt aus der Gruppe bestehend aus Instant-Kakao-Getränke, Instant-Tee-Getränke und Instant-Kaffeegetränke, Fleischprodukte vorzugsweise ausgewählt aus der Gruppe bestehend aus Schinken, Frischwurst- oder Rohwurstzubereitungen und gewürzte oder marinierte Frisch- oder Pökelfleischprodukte, Eier oder Eiprodukte vorzugsweise ausgewählt aus der Gruppe bestehend aus Trockenei, Eiweiß und Eigelb, Getreideprodukte vorzugsweise ausgewählt aus der Gruppe bestehend aus Frühstückscerealien, Müsliriegel und vorgegarte Fertigreis-Produkte, Milchprodukte vorzugsweise ausgewählt aus der Gruppe bestehend aus Milchgetränke, Milcheis, Joghurt, Kefir, Frischkäse, Weichkäse, Hartkäse, Trockenmilchpulver, Molke, Butter, Buttermilch und teilweise oder ganz hydrolisierte Milchproteinhaltige Produkte, Produkte aus Sojaprotein oder anderen Sojabohnen-Fraktionen vorzugsweise ausgewählt aus der Gruppe bestehend aus Sojamilch und daraus gefertigte Produkte, Sojalecithin-haltige Zubereitungen, fermentierte Produkte wie Tofu oder Tempe oder daraus gefertigte Produkte und Sojasoßen, Fruchtzubereitungen vorzugsweise ausgewählt aus der Gruppe bestehend aus Konfitüren, Fruchteis, Fruchtsoßen und Fruchtfüllungen, Gemüsezubereitungen vorzugsweise ausgewählt aus der Gruppe bestehend aus Ketchup, Soßen, Trockengemüse, Tiefkühlgemüse, vorgegarte Gemüse, in Essig eingelegte Gemüse und eingekochte Gemüse, Knabberartikel vorzugsweise ausgewählt aus der Gruppe bestehend aus gebackenen oder frittierten Kartoffelchips oder Kartoffelteigprodukte, Brotteigprodukte und Extrudate auf Mais- oder Erdnussbasis, Produkte auf Fett- und Ölbasis oder Emulsionen derselben vorzugsweise ausgewählt aus der Gruppe bestehend aus Mayonnaise, Remoulade, Dressings und Würzzubereitungen, sonstige Fertiggerichte und Suppen vorzugsweise ausgewählt aus der Gruppe bestehend aus Trockensuppen, Instant-Suppen, vorgegarte Suppen, Gewürze, Würzmischungen und Aufstreuwürzungen (englisch: Seasonings), die beispielsweise im Snackbereich Anwendung finden.

Gemäß einer bevorzugten erfindungsgemäßen Verwendung werden ein oder mehrere erfindungsgemäße Verbindungen der Formel (I) und/oder Formel (Ia) als Riech- und/oder Aromastoffe bevorzugt mit moschusartigen Noten, weiter bevorzugt in erfindungsgemäßen Riech- und/oder Aromastoffkompositionen oder erfindungsgemäßen parfümierten oder aromatisierten Produkten verwendet.

Gemäß einer weiteren bevorzugten Ausgestaltung werden ein, zwei, drei, vier oder mehrere erfindungsgemäße Verbindungen der Formel (I) und/oder Formel (Ia) zum Vermitteln, Modifizieren und/oder Verstärken eines Geruchs oder Geschmacks mit einer, mehreren oder sämtlichen moschusartigen Noten verwendet.

Gemäß einer weiteren bevorzugten Ausgestaltung werden eine, zwei, drei, vier oder mehrere erfindungsgemäße Verbindungen der Formel (I) und/oder Formel (Ia) als Booster verwendet, bevorzugt in einer erfindungsgemäßen Riech-und/oder Aromastoffkomposition sowie deren bevorzugten Ausgestaltungen.

Gemäß einer weiteren bevorzugten Ausgestaltung werden eine, zwei, drei, vier oder mehrere erfindungsgemäße Verbindungen der Formel (I) und/oder Formel (Ia) zur Erhöhung der Substantivität weiterer Riech- und/oder Aromastoffe, bevorzugt in der Komponente (b) einer erfindungsgemäßen Riech- und/oder Aromastoffkomposition sowie auch deren bevorzugten Ausgestaltungen, auf Haut, Haare und/oder Textilfasern verwendet.

Gemäß einer weiteren bevorzugten Ausgestaltung werden eine, zwei, drei, vier oder mehrere erfindungsgemäße Verbindungen der Formel (I) und/oder Formel (Ia) zur Erhöhung der Retention weiterer Riech- und/oder Aromastoffe, bevorzugt in der Komponente (b) einer erfindungsgemäßen Riech- und/oder Aromastoffkomposition sowie auch deren bevorzugten Ausgestaltungen, verwendet.

Gemäß einer weiteren bevorzugten Ausgestaltung werden eine, zwei, drei, vier oder mehrere erfindungsgemäße Verbindungen der Formel (I) und/oder Formel (Ia) verwendet, um einer erfindungsgemäßen Riech- und/oder Aromastoffkomposition, insbesondere auch deren bevorzugten Ausgestaltungen, (Aus)Strahlung, Abrundung und/oder Harmonie zu verleihen und/oder vorhandene Geruchs- und/oder Geschmacksnoten in einer erfindungsgemäßen Riech- und/oder Aromastoffkomposition, insbesondere auch deren bevorzugten Ausgestaltungen, zu verstärken.

Die Herstellung erfindungsgemäßer Verbindungen der Formel (I) können mittels an sich bekannter Reaktionen und Verfahren (Jerry March, Advanced Organic Chemistry: Reactions, Mechanisms and Structure, 4. Ed., John Wiley and Sons, 1992 und Richard C. Larock, Comprehensive Organic Transformations: A Guide to functional Group Preparations, VCH Verlag, 1989) mit einem oder mehreren folgenden Schritten erfolgen:
a) Epoxidation einer Verbindung der Formel (II) zu einer entsprechenden Verbindung der Formel (III) 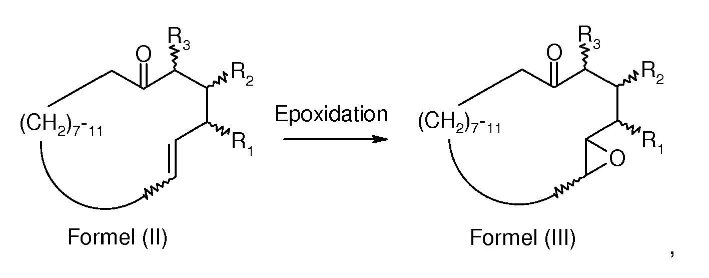 wobei R₁, R₂ und R₃ die oben dargestellten Bedeutungen haben,
b) anschließende Reduktion der entsprechenden Verbindung der Formel (III) zu einer entsprechenden Verbindung der Formel (IV) 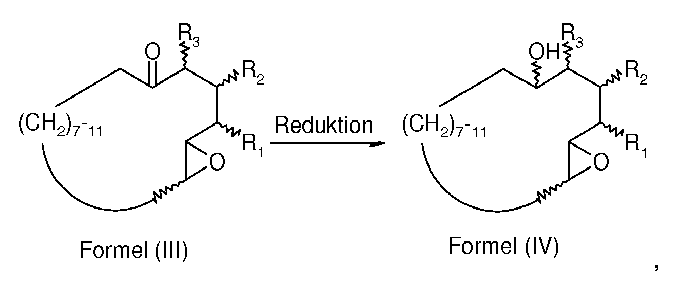 wobei R₁, R₂ und R₃ die oben dargestellten Bedeutungen haben,
c) anschließende Cyclisierung der entsprechenden Verbindung der Formel (IV) zu einer entsprechenden Verbindung der Formel (V) 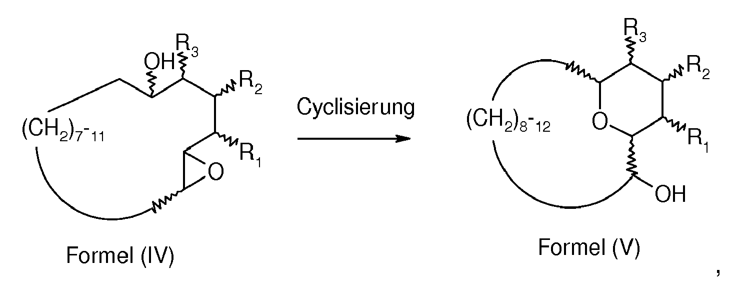 wobei R₁, R₂ und R₃ die oben dargestellten Bedeutungen haben
   und
d) anschließende Oxidation der entsprechenden Verbindung der Formel (V) zu einer entsprechenden Verbindung der Formel (I) 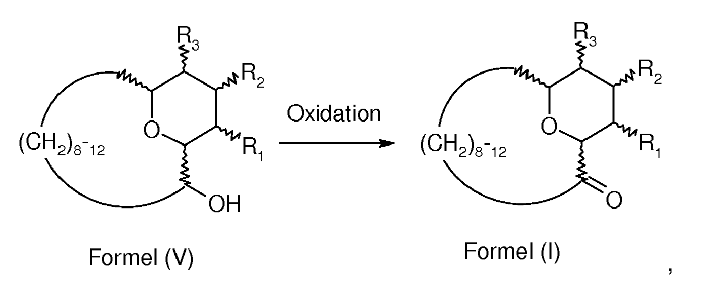
wobei R₁, R₂ und R₃ die oben dargestellten Bedeutungen haben.

Die Herstellung der erfindungsgemäßen Verbindungen der Formel (I) kann durch entsprechende Anpassung an sich bekannter Reaktionen und Verfahren (Jerry March, Advanced Organic Chemistry: Reactions, Mechanisms and Structure, 4. Ed., John Wiley and Sons, 1992 und Richard C. Larock, Comprehensive Organic Transformations: A Guide to functional Group Preparations, VCH Verlag, 1989) erfolgen.

Im Folgenden wird die Herstellung von 17-Oxa-bicyclo[11.3.1]heptadecan-2-on und 14-Methyl-16-Oxa-bicyclo[10.3.1]hexadecan-2-on als exemplarische Beispiele für die Herstellung einer Verbindung der Formel (I) dargestellt:

### 17-Oxa-bicyclo[11.3.1]heptadecan-2-on:

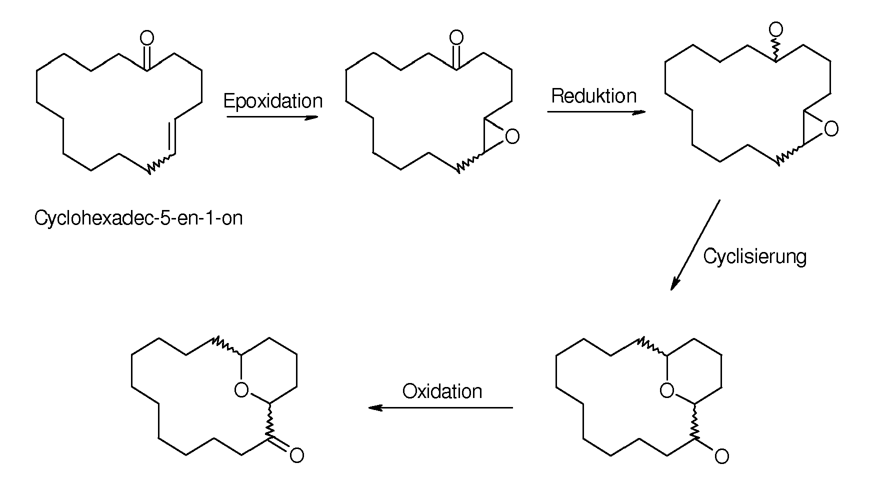

Analog zu dieser Herstellung können aus Cyclotetradec-5-en-1-on, Cyclopentadec-5-en-1-on, Cycloheptadec-5-en-1-on und Cyclooctadec-5-en-1-on die entsprechenden weiteren Verbindungen der Formel (I) hergestellt werden.

### 14-Methyl-16-Oxa-bicyclo[10.3.1]hexadecan-2-on:

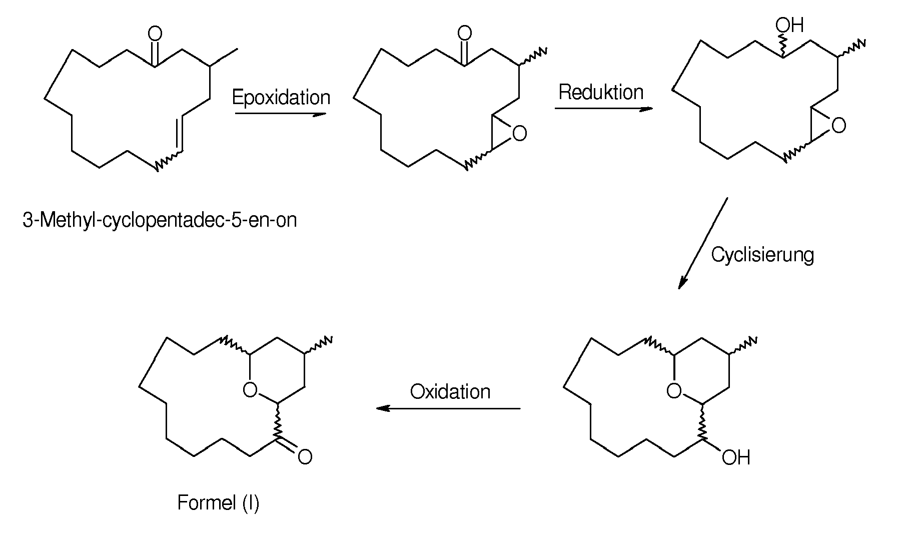

Analog zu dieser Herstellung können aus 2-Methyl-cyclopentadec-5-en-1-on, 3-Methyl-cyclopentadec-5-en-1-on und 4-Methyl-cyclopentadec-5-en-1-on die entsprechenden weiteren Verbindungen der Formel (I) hergestellt werden.

Die Herstellung erfindungsgemäßer Verbindungen der Formel (Ia) können ebenfalls mittels an sich bekannter Reaktionen und Verfahren (Jerry March, Advanced Organic Chemistry: Reactions, Mechanisms and Structure, 4. Ed., John Wiley and Sons, 1992 und Richard C. Larock, Comprehensive Organic Transformations: A Guide to functional Group Preparations, VCH Verlag, 1989) mit einem oder mehreren folgenden Schritten erfolgen:
a) Reduktion einer Verbindung der Formel (IIa) zu einer entsprechenden Verbindung der Formel (IIIa) 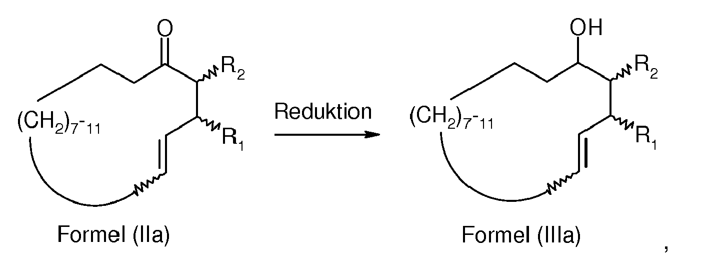 wobei R₁ und R₂ die oben dargestellten Bedeutungen haben,
b) anschließende Epoxidation der entsprechenden Verbindung der Formel (III) zu einer entsprechenden Verbindung der Formel (IVa) 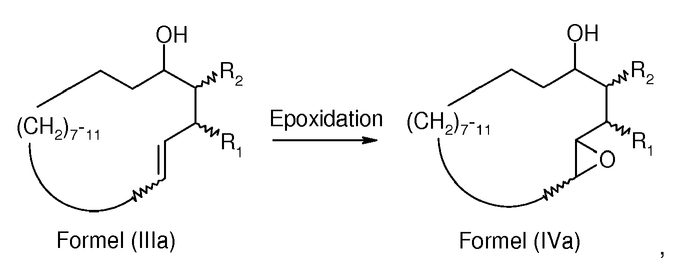 wobei R₁ und R₂ die oben dargestellten Bedeutungen haben,
c) anschließende Cyclisierung der entsprechenden Verbindung der Formel (IVa) zu einer entsprechenden Verbindung der Formel (Va) 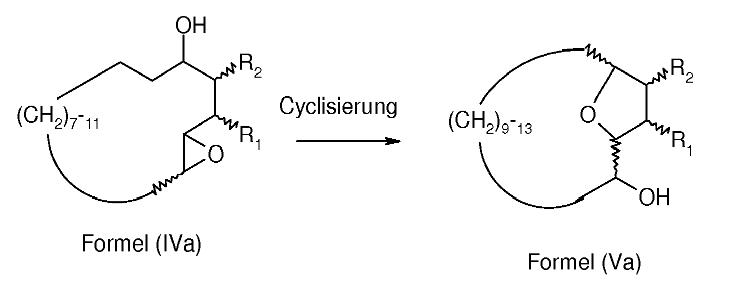 wobei R₁ und R₂ die oben dargestellten Bedeutungen haben
   und
d) anschließende Oxidation der entsprechenden Verbindung der Formel (Va) zu einer entsprechenden Verbindung der Formel (Ia) 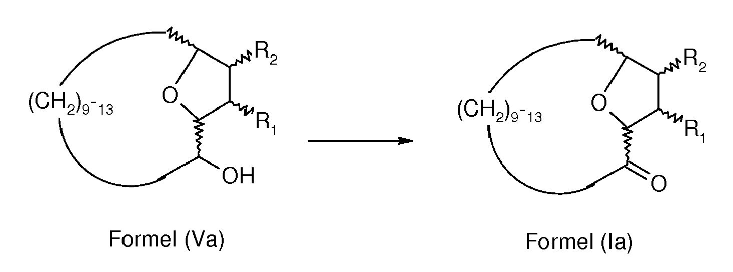
wobei R₁ und R₂ die oben dargestellten Bedeutungen haben.

Die Herstellung der erfindungsgemäßen Verbindungen der Formel (Ia) können durch entsprechende Anpassung an sich bekannter Reaktionen und Verfahren (Jerry March, Advanced Organic Chemistry: Reactions, Mechanisms and Structure, 4. Ed., John Wiley and Sons, 1992 und Richard C. Larock, Comprehensive Organic Transformations: A Guide to functional Group Preparations, VCH Verlag, 1989) erfolgen.

Im Folgenden wird die Herstellung von 16-Oxa-bicyclo[11.2.1]hexadecan-2-on und 15-Methyl-16-Oxa-bicyclo[11.2.1]hexadecan-2-on als exemplarische Beispiele für die Herstellung einer Verbindung der Formel (Ia) dargestellt:

### 16-Oxa-bicyclo[11.2.1]hexadecan-2-on:

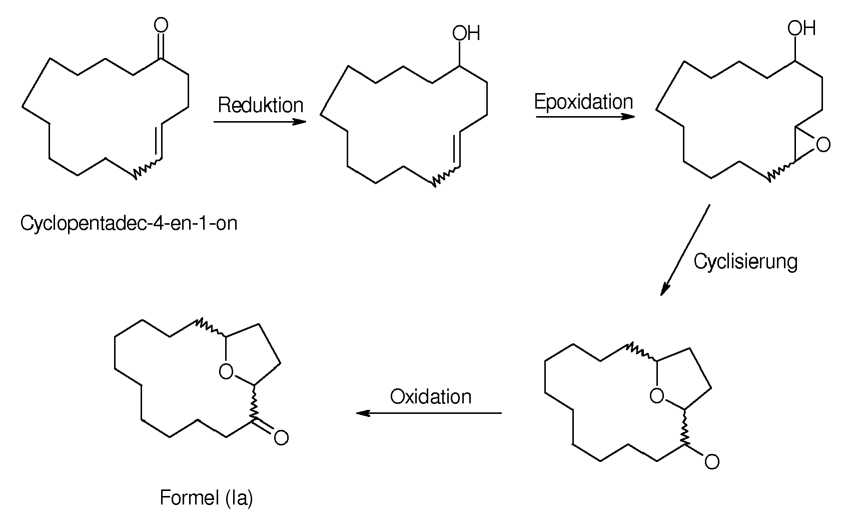

Analog zu dieser Herstellung können aus Cyclotetradec-4-en-1-on, Cyclohexadec-4-en-1-on Cycloheptadec-4-en-1-on und Cyclooctadec-4-en-1-on die entsprechenden weiteren Verbindungen der Formel (Ia) hergestellt werden.

### 15-Methyl-16-Oxa-bicyclo[11.2.1]hexadecan-2-on:

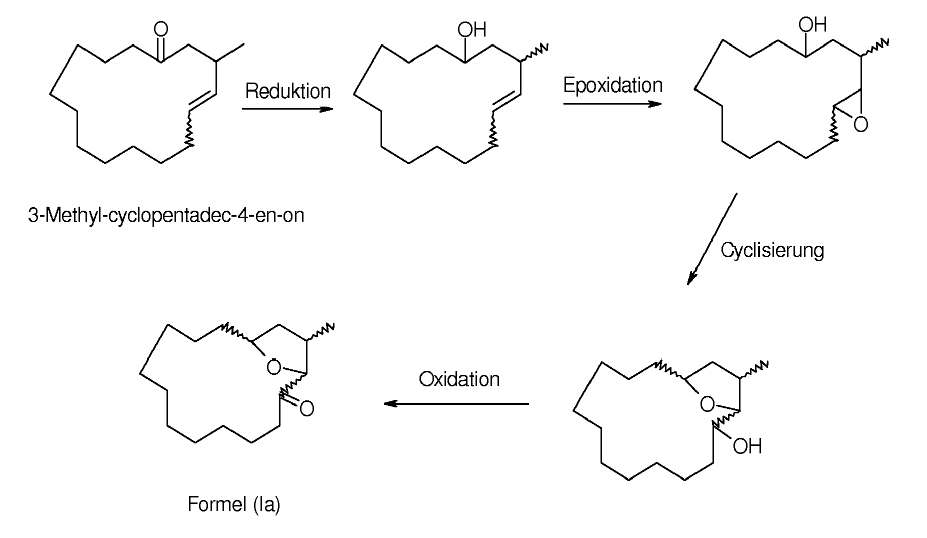

Analog zu dieser Herstellung kann aus 2-Methyl-cyclopentadec-4-en-1-on die entsprechenden weiteren Verbindungen der Formel (Ia) hergestellt werden.

Alternativ können Verbindungen der Formel (I) und der Formel (Ia) bei Auswahl geeigneter Edukte und Anpassung eines oben beschriebenen Herstellungsverfahrens gleichzeitig hergestellt werden.

Ein, zwei, drei, vier oder mehrere erfindungsgemäße Verbindungen der Formel (I) und Formel (Ia) oder ein oder mehrere erfindungsgemäße Riech- und/oder Aromastoffkompositionen, die ein, zwei, drei, vier oder mehrere erfindungsgemäße Verbindungen der Formel (I) und/oder der Formel (Ia) enthalten, können üblicherweise in konzentrierter Form, in Lösungen oder in oben beschriebener modifizierter Form verwendet werden für die Herstellung von erfindungsgemäßen parfümierten Kosmetik- oder Haushaltsprodukten, wobei diese Produkte vorzugsweise ausgewählt werden, aus der Gruppe bestehend aus:

Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmitteln vorzugsweise ausgewählt aus der Gruppe bestehend aus Fußbodenreinigern, Fensterglasreinigern, Geschirrspülmittel, Bad- oder Sanitärreiniger, Scheuermilch, festen oder flüssigen WC-Reinigern, pulver- oder schaumförmigen Teppichreinigern, Textilerfrischer, Bügelhilfen, flüssige Waschmitteln, pulverförmige Waschmitteln, Wäschevorbehandlungsmittel, Wäscheweichspüler, Waschseifen und Waschtabletten, Desinfektionsmittel, Oberflächendesinfektionsmittel, Luftverbesserer in flüssiger, gelartiger oder auf einem festen Träger aufgebrachter Form, Aerosolsprays, Wachse und Polituren vorzugsweise ausgewählt aus der Gruppe bestehend aus Möbelpolituren, Fußbodenwachsen und Schuhcremes, Körperpflegemittel vorzugsweise ausgewählt aus der Gruppe bestehend aus festen und flüssigen Seifen, Duschgelen, Shampoos, Rasierseifen, Rasierschäumen, Badeölen und kosmetischen Emulsionen vom Öl-in-Wasser-, vom Wasser-in-Öl- und vom Wasser-in-Öl-in-Wasser-Typ vorzugsweise ausgewählt aus der Gruppe bestehend aus Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten vorzugsweise ausgewählt aus der Gruppe bestehend aus Haarsprays, Haargelen, festigenden Haarlotionen, Haarspülungen, permanenten und semipermanenten Haarfärbemittel, Haarverformungsmittel vorzugsweise ausgewählt aus der Gruppe bestehend aus Kaltwellen- und Haarglättungsmittel; Haarwässern, Haarcremes und -lotionen, Deodorantien und Antiperspirantien vorzugsweise ausgewählt aus der Gruppe bestehend aus Achselsprays, Roll-ons, Deosticks und Deocremes, Produkte der dekorativen Kosmetik vorzugsweise ausgewählt aus der Gruppe bestehend aus Lidschatten, Nagellacke, Make-ups, Lippenstifte und Mascara, Kerzen, Lampenöle, Räucherstäbchen, Insektizide, Repellentien und Treibstoffe.

Die erfindungsgemäßen Verbindungen der Formel (I) und/oder der Formel (Ia) können in aromatisierte oder zu aromatisierende Artikel eingearbeitet werden, insbesondere in der Ernährung, der Mundpflege oder dem Genuss dienende Zubereitungen, Halbfertigmischungen und Würzmischungen.

Die erfindungsgemäßen der Ernährung oder dem Genuss dienenden Zubereitungen werden vorzugsweise ausgewählt aus der Gruppe bestehend aus:
Backwaren vorzugsweise ausgewählt aus der Gruppe bestehend aus Brot, Trockenkekse, Kuchen und sonstiges Gebäck,
Süßwaren vorzugsweise ausgewählt aus der Gruppe bestehend aus Schokolade, Schokoladenriegelprodukte, sonstige Riegelprodukte, Fruchtgummi, Hart- und Weichkaramellen und Kaugummi,
alkoholische oder nicht-alkoholische Getränke vorzugsweise ausgewählt aus der Gruppe bestehend aus Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte und Frucht- oder Gemüsesaftzubereitungen,
Instantgetränke vorzugsweise ausgewählt aus der Gruppe bestehend aus Instant-Kakao-Getränke, Instant-Tee-Getränke und Instant-Kaffeegetränke,
Fleischprodukte vorzugsweise ausgewählt aus der Gruppe bestehend aus Schinken, Frischwurst- oder Rohwurstzubereitungen und gewürzte oder marinierte Frisch- oder Pökelfleischprodukte,
Eier oder Eiprodukte vorzugsweise ausgewählt aus der Gruppe bestehend aus Trockenei, Eiweiß und Eigelb,
Getreideprodukte vorzugsweise ausgewählt aus der Gruppe bestehend aus Frühstückscerealien, Müsliriegel und vorgegarte Fertigreis-Produkte,
Milchprodukte vorzugsweise ausgewählt aus der Gruppe bestehend aus Milchgetränke, Milcheis, Joghurt, Kefir, Frischkäse, Weichkäse, Hartkäse, Trockenmilchpulver, Molke, Butter, Buttermilch und teilweise oder ganz hydrolisierte Milchprotein-haltige Produkte,
Produkte aus Sojaprotein oder anderen Sojabohnen-Fraktionen vorzugsweise ausgewählt aus der Gruppe bestehend aus Sojamilch und daraus gefertigte Produkte, Sojalecithin-haltige Zubereitungen, fermentierte Produkte wie Tofu oder Tempe oder daraus gefertigte Produkte und Sojasoßen,
Fruchtzubereitungen vorzugsweise ausgewählt aus der Gruppe bestehend aus Konfitüren, Fruchteis, Fruchtsoßen und Fruchtfüllungen,
Gemüsezubereitungen vorzugsweise ausgewählt aus der Gruppe bestehend aus Ketchup, Soßen, Trockengemüse, Tiefkühlgemüse, vorgegarte Gemüse, in Essig eingelegte Gemüse und eingekochte Gemüse,
Knabberartikel vorzugsweise ausgewählt aus der Gruppe bestehend aus gebackenen oder frittierten Kartoffelchips oder Kartoffelteigprodukte, Brotteigprodukte und Extrudate auf Mais- oder Erdnussbasis,
Produkte auf Fett- und Ölbasis oder Emulsionen derselben vorzugsweise ausgewählt aus der Gruppe bestehend aus Mayonnaise, Remoulade, Dressings und Würzzubereitungen,
sonstige Fertiggerichte und Suppen vorzugsweise ausgewählt aus der Gruppe bestehend aus Trockensuppen, Instant-Suppen, vorgegarte Suppen, Gewürze, Würzmischungen und Aufstreuwürzungen (englisch: Seasonings), die beispielsweise im Snackbereich Anwendung finden.

Erfindungsgemäße aromatisierte Produkte können insbesondere als Halbfertigware zur Herstellung weiterer der Ernährung oder dem Genuss dienenden Zubereitungen dienen, bevorzugt in sprühgetrockneter Form. Erfindungsgemäße aromatisierte Halbfertigprodukte können auch bevorzugt in Form von Kapseln, Tabletten, bevorzugt nichtüberzogenen sowie überzogenen Tabletten (z.B. magensaftresistente Überzüge), Dragees, Granulaten, Pellets, Feststoffmischungen, Dispersionen in flüssigen Phasen, Emulsionen, Pulver, Lösungen, Pasten oder andere schluck- oder kaubare Zubereitungen als Nahrungsergänzungsmittel vorliegen.

Die erfindungsgemäßen der Mundpflege dienenden aromatisierten Produkte werden bevorzugt ausgewählt aus Mund- und/oder Zahnpflegemittel, wie Zahnpasten, Zahngele, Zahnpulver, Mundwässer, Kaugummis und andere Mundpflegemittel.

Übliche Wirk-, Grund-, Hilfs- und Zusatzstoffe für der Ernährung, der Mundpflege oder dem Genuss dienende erfindungsgemäße aromatisierte Produkte können in Mengen von 5 bis 99,999999 Gew.-%, vorzugsweise 10 bis 80 Gew.-%, bezogen auf das Gesamtgewicht des Produktes, enthalten sein. Ferner können die aromatisierten Produkte Wasser in einer Menge bis zu 99,999999 Gew.-%, vorzugsweise 5 bis 80 Gew.-%, bezogen auf das Gesamtgewicht des Produktes, aufweisen.

Erfindungsgemäße aromatisierten Produkte, umfassend erfindungsgemäße Verbindungen der Formel (I) und/oder der Formel (Ia), werden gemäß einer bevorzugten Ausgestaltung hergestellt, indem eine oder mehrere erfindungsgemäße Verbindungen der Formel (I) und/oder der Formel (Ia), üblicherweise als feste Substanz, als Lösung (z. B. in Ethanol, Wasser oder 1,2-Propylenglycol) oder in Form eines Gemisches mit einem festen oder flüssigen Trägerstoff (z.B. Maltodextrin, Stärke, Silicagel), weiteren Riech- und/oder Aromastoffen und gegebenenfalls weiteren Hilfsmitteln und/oder Stabilisatoren (vorzugsweise natürliche oder künstliche Polysaccharide und/oder Pflanzengummen wie modifizierte Stärken oder Gummi Arabicum) in einer der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße aromatisierte Halbfertigprodukte (Halbfertigwaren) überführt werden.

Die erfindungsgemäßen sprühgetrockneten festen aromatisierten Produkte sind als Halbfertigwaren besonders gut zur Herstellung von weiteren erfindungsgemäßen aromatisierten Produkten geeignet. In den bevorzugten erfindungsgemä-ßen sprühgetrockneten festen aromatisierten Produkten sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform erfindungsgemäßer aromatisierter Produkte werden zu deren Herstellung zunächst ein, zwei, drei, vier oder mehrere erfindungsgemäße Verbindungen der Formel (I) und/oder Formel (Ia) und gegebenenfalls weiteren Bestandteilen des erfindungsgemäßen aromatisierten Produktes, bevorzugt weitere ein, zwei, drei oder mehrere Riech- und/oder Aromastoffe, in Emulsionen, Liposomen, z.B. ausgehend von Phosphatidylcholin, Microsphären, Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für ein erfindungsgemäßes aromatisiertes Produkte werden eine oder mehrere Verbindungen Formel (I) und/oder Formel (Ia) zunächst mit einem oder mehreren Komplexbildnern, beispielsweise mit Cyclodextrinen oder Cyclodextrinderivaten, bevorzugt α- oder β-Cyclodextrin, komplexiert und in dieser komplexierten Form eingesetzt.

Bevorzugt ist eine erfindungsgemäße Zubereitung, bei der die Matrix so gewählt ist, dass eine, zwei, drei, vier oder mehrere erfindungsgemäße Verbindungen der Formel (I) und/oder Formel (Ia) verzögert von der Matrix freigegeben werden, so dass eine langanhaltende sensorische Wirkung erzielt wird. Besonders bevorzugt ist insoweit eine Fett-, Wachs-, Polysaccharid- oder Proteinmatrix.

Als weitere Bestandteile für erfindungsgemäße der Ernährung oder dem Genuss dienende Zubereitungen können übliche Grund-, Hilfs- und Zusatzstoffe für Nahrungs- oder Genussmittel verwendet werden, vorzugsweise werden ein, zwei, drei, vier oder mehrere dieser Bestandteile ausgewählt aus der Gruppe bestehend aus Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für die Mundpflege dienende aromatisierte Produkte), die eine oder mehrere erfindungsgemäße Verbindungen der Formel (I) und/oder Formel (Ia) und/oder erfindungsgemäße Riech- und/oder Aromastoffe enthalten, umfassen im Allgemeinen ein abrasives System (Schleif- oder Poliermittel), wobei ein, zwei, drei oder mehrere Bestandteile des abrasiven Systems bevorzugt ausgewählt werden aus der Gruppe bestehend aus Kieselsäuren, Calciumcarbonate, Calciumphosphate, Alumiuniumoxide und/oder Hydroxylapatite, oberflächenaktive Substanzen, wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide, wie Guanosinmonophosphat, Adenosinmonophosphat oder andere Stoffe wie Natriumglutamat oder 2-Phenoxypropionsäure), Kühlwirkstoffen, wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für die Mundpflege dienende aromatisierte Produkte), welche die Verbindung der Formel (I) und/oder Formel (Ia) enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Bevorzugt können erfindungsgemäße parfümierte oder aromatisierte Produkte neben ein oder mehreren erfindungsgemäßen Verbindungen der Formel (I) und/oder Formel (Ia) eine, zwei, drei, vier oder mehrere erfindungsgemäße Riech- und/oder Aromastoffkompositionen enthalten, um den Geschmack und/oder Geruch des parfümierten oder aromatisierten Produktes weiter abzurunden und/oder zu verfeinern. Geeignete (zusätzliche) erfindungsgemäße Riech- und/oder Aromastoffkompositionen enthalten vorzugsweise ein oder mehrere synthetische, natürliche und/oder naturidentische Aroma-, Riech-und/oder Geschmacksstoffe sowie weiter vorzugsweise ein oder mehrere geeignete Hilfs- und Trägerstoffe.

Nachfolgend wird die Erfindung anhand von Beispielen näher erläutert. Sofern nicht anders angegeben, beziehen sich alle Angaben auf das Gewicht.

### Beispiel 1: Herstellung von 17-Oxa-bicyclo[11.3.1]heptadecan-2-on als Verbindung der Formel (I)

### 1. Stufe: Epoxidation

In einen 500 ml Rührwerk wurden 23,6 g (0,1 mol) Cyclohexadec-5-en-1-on in 250 ml Methylenchlorid, mit 25 g Natriumacetat vorgelegt. Anschließend wurde unter Rühren und Kühlen bei 18-25°C innerhalb von 2 Stunden 23,4 g (0,12 mol) Peressigsäure 38%ig zugetropft. Danach wurde 4 Stunden bei Raumtemperaturnachgerührt. Die Reaktionslösung wurde mit Wasser Eisen(II)sulfatlsg. Und Sodalsg. gewaschen und eingeengt. Rohausbeute: 24,3 g.

GC-Auswertung (20m ZB-WAX, Innendurchmesser 0,18µm / 60-9-220°C Kaltaufgabesystem)

Das Rohprodukt wurde ohne weitere Reinigung in der 2. Stufe umgesetzt.

### 2. Stufe: Reduktion

In einen 250 ml Rührwerk wurden 24,3 g Rohprodukt 1. Stufe in 100 ml Methanol unter Rühren und Kühlen bei 20°C mit einer Lösung aus 5 g Wasser, 20 mg NaOH und 1,3 g Natriumboranat versetzt. Danach wurde 4 h bei 20°C nachgerührt. Zur Aufarbeitung wurde das Methanol am Rotationsverdampfer entfernt, der Rückstand mit 100 g Toluol und 100 g Wasser versetzt. Nach Trennung der Phasen wurde die organische Phase einmal mit Wasser gewaschen und das Toluol entfernt. Rohausbeute: 24,3 g.

GC-Auswertung (20m ZB-WAX, Innendurchmesser 0,18µm / 60-9-220°C Kaltaufgabesystem)

Das Rohprodukt wurde ohne weitere Reinigung in der 3. Stufe umgesetzt.

### 3. Stufe: Cyclisierung

In einen 250 ml Rührwerk wurden 24,3 g Rohprodukt 2. Stufe in 200 ml Toluol unter Rühren bei 20°C mit 25 mg p-Toluolsulfonsäure versetzt. Danach wurde 4 h bei 20°C nachgerührt. Zur Aufarbeitung wurde das Reaktionsgemisch mit Natriumhydrogencarbonatlsg. gewaschen. Nach Trennung der Phasen wurde die organische Phase einmal mit Wasser gewaschen und das Toluol entfernt. Rohausbeute: 24,2 g.

GC-Auswertung (20m ZB-WAX, Innendurchmesser 0,18µm / 60-9-220°C Kaltaufgabesystem)

Das Rohprodukt wurde ohne weitere Reinigung in der 4. Stufe umgesetzt.

### 4. Stufe: Oxidation

In einen 2 I Rührwerk wurden 24,2 g Rohprodukt 3. Stufe in 1000 ml Methylenchlorid gelöst und unter Rühren bei 20-25°C mit 56,8 g (0,15 mol) Pyridiniumdichromat versetzt. Danach wurde 4 h bei 20°C nachgerührt. Zur Aufarbeitung wurde das Reaktionsgemisch über Kieselgel filtriert, mit verd. Schwefelsäure und Natriumhydrogencarbonatlsg. gewaschen und das Methylenchlorid entfernt. Rohausbeute: 23,8 g (ca. 1:1 E/Z-Isomere).

GC-Auswertung (20m ZB-WAX, Innendurchmesser 0,18µm / 60-9-220°C Kaltaufgabesystem)

Das Rohprodukt wurde über Säulenchromatographie gereinigt.

Es wurden die spektroskopischen Daten von hergestellten 17-Oxa-bicyclo[11.3.1]heptadecan-2-on ermittelt. Die Daten der E/Z Isomere sind nachfolgend angegeben.

### 17-Oxa-bicyclo[11.3.1]heptadecan-2-on Z-Isomer

¹H-NMR (CDCl₃, 400 MHZ, TMS= 0): 3,8 (J=11.4, J=2,4, 1 H); 3,4(J=11.3, J=9,8, J=2,2, 1 H); 2,8(1 H); 2,2 (1 H); 1,76-1,94(3H); 1,20-1,75(21 H).
¹³C-NMR (CDCl₃, 100 MHz): 212,8(s), 81,9(d), 77,5(d), 38,4(t), 35,2(t), 31,9(t), 26,9(t), 26,7(t), 26,3(t), 25,8(t), 25,7(t),. 24,9 (t), 24,2(t), 23,34(t), 23,32(t), 23,2(t).
MS: m/z (%) = 252 (14, M⁺), 224 (8), 111 (12), 98 (32), 85 (36), 67 (42), 55 (100), 41 (96), 29 (37).

### Geruchsbeschreibung Z-Isomer: schwacher Moschusgeruch, leicht lederiganimalisch

**17-Oxa-bicyclo[11.3.1]heptadecan-2-on E-Isomer**
¹H-NMR (CDCl₃, 400 MHZ, TMS= 0): 4,2(1H); 3,3(J=9,6, J=4,1, J=2,3, 1 H); 3,1 (J=17.2, J=11,0, J=5,6, 1H); 2,2(J=17.2, J=5.0, J=0.7, 1H); 1,8-2,1 (1 H); 1,1-1,8(23H).
¹³C-NMR (CDCl₃, 100 MHz): 212,9 (s), 78,2(d), 72,5(d), 36,5(t), 34,4(t), 31,4 (t), 25,9(t), 25,44(t), 25,43(t), 24,6(t), 24,0(t), 23,9(t), 23,8(t), 23,1 (t), 20,5(t), 19,7(t).
MS: m/z (%) = 252 (20, M⁺), 224 (10), 111 (12), 98 (28), 85 (34), 67 (43), 55 (100), 41 (90), 29 (33).

### Geruchsbeschreibung E-Isomer: sehr starker sauberer Moschusgeruch, interessante feine erogene Duftnote, sehr natürlicher Geruchseindruck.

### Beispiel 2: Herstellung von 14-Methyl-16-Oxa-bicyclo[10.3.1]hexadecan-2-on als Verbindung der Formel (I)

### 1. Stufe: Epoxidation

In einen 500 ml Rührwerk wurden 23,6 g (0,1 mol) 3-Methyl-cyclopentadec-5-en-1-on (angereichert durch Destillation aus kommerzielle erhältlichen 3-Methylcyclopentadec-5-en-1-on 82%ig) in 250 ml Methylenchlorid, mit 25 g Natriumacetat vorgelegt. Anschließend wurde unter Rühren und Kühlen bei 18-25°C innerhalb von 2 Stunden 23,4 g (0,12 mol) Peressigsäure 38%ig zugetropft. Danach wurde 4 Stunden bei Raumtemperaturnachgerührt. Die Reaktionslösung wurde mit Wasser Eisen(II)sulfatlösung Und Sodalösung gewaschen und eingeengt. Rohausbeute: 24,9 g.

GC-Auswertung (20m ZB-WAX, Innendurchmesser 0,18µm / 60-9-220°C Kaltaufgabesystem)

Das Rohprodukt wurde ohne weitere Reinigung in der 2. Stufe umgesetzt.

### 2. Stufe: Reduktion

In einen 250 ml Rührwerk wurden 24,9 g Rohprodukt 1. Stufe in 100 ml Methanol unter Rühren und Kühlen bei 20°C mit einer Lösung aus 5 g Wasser, 20 mg NaOH und 1,3 g Natriumboranat versetzt. Danach wurde 4 h bei 20°C nachgerührt. Zur Aufarbeitung wurde das Methanol am Rotationsverdampfer entfernt, der Rückstand mit 100 g Toluol und 100 g Wasser versetzt. Nach Trennung der Phasen wurde die organische Phase einmal mit Wasser gewaschen und das Toluol entfernt. Rohausbeute: 24,6 g.

GC-Auswertung (20m ZB-WAX, Innendurchmesser 0,18µm / 60-9-220°C Kaltaufgabesystem)

Das Rohprodukt wurde ohne weitere Reinigung in der 3. Stufe umgesetzt.

### 3. Stufe: Cyclisierung

In einen 250 ml Rührwerk wurden 24,6 g Rohprodukt 2. Stufe in 200 ml Toluol unter Rühren bei 20°C mit 25 mg p-Toluolsulfonsäure versetzt. Danach wurde 4 h bei 20°C nachgerührt. Zur Aufarbeitung wurde das Reaktionsgemisch mit Natriumhydrogencarbonatlsg. gewaschen. Nach Trennung der Phasen wurde die organische Phase einmal mit Wasser gewaschen und das Toluol entfernt. Rohausbeute: 24,4 g.

GC-Auswertung (20m ZB-WAX, Innendurchmesser 0,18µm / 60-9-220°C Kaltaufgabesystem)

Das Rohprodukt wurde ohne weitere Reinigung in der 4. Stufe umgesetzt.

### 4. Stufe: Oxidation

In einen 2 I Rührwerk wurden 24,4 g Rohprodukt 3. Stufe in 1000 ml Methylenchlorid gelöst und unter Rühren bei 20-25°C mit 56,8 g (0,15 mol) Pyridiniumdichromat versetzt. Danach wurde 4 h bei 20°C nachgerührt. Zur Aufarbeitung wurde das Reaktionsgemisch über Kieselgel filtriert, mit verd. Schwefelsäure und Natriumhydrogencarbonatlsg. gewaschen und das Methylenchlorid entfernt. Rohausbeute: 23,6 g (ca. 1:1 E/Z-Isomere).

GC-Auswertung (20m ZB-WAX, Innendurchmesser 0,18µm / 60-9-220°C Kaltaufgabesystem)

Das Rohprodukt wurde über Säulenchromatographie gereinigt.

Es wurden die spektroskopischen Daten von hergestellten 14-Methyl-16-Oxa-bicyclo[10.3.1]hexadecan-2-on ermittelt. Die Daten Isomere sind nachfolgend angegeben.

### 14-Methyl-16-Oxa-bicyclo[10.3.1]hexadecan-2-on

### Isomer 1 Index 1862

MS: m/z (%) = 252 (96, M⁺), 180 (57), 99 (67), 97 (60), 81 (73), 71 (65), 68 (64), 55 (100), 41 (71).

### Geruchsbeschreibung: angenehmer weicher moschusartiger Geruch

### 14-Methyl-16-Oxa-bicyclo[10.3.1]hexadecan-2-on

### Isomer 2 Index 1868

MS: m/z (%) = 252 (86, M⁺), 180 (60), 99 (69), 97 (62), 81 (76), 71 (66), 68 (66), 55 (100), 41 (72).

### Geruchsbeschreibung: angenehmer weicher moschusartiger Geruch

### 14-Methyl-16-Oxa-bicyclo[10.3.1]hexadecan-2-on

### Isomer 3 Index 1891

MS: m/z (%) = 252 (75, M⁺), 180 (53), 99 (74), 97 (60), 81 (72), 71 (65), 68 (63), 55 (100), 41 (73).

### Geruchsbeschreibung: angenehmer weicher moschusartiger Geruch

### 14-Methyl-16-Oxa-bicyclo[10.3.1]hexadecan-2-on

### Isomer 4 Index 1907

MS: m/z (%) = 252 (77, M⁺), 180 (56), 99 (75), 97 (62), 81 (72), 71 (65), 68 (63), 55 (100), 41 (70).

### Geruchsbeschreibung: angenehmer weicher moschusartiger Geruch

### Beispiel 3: Herstellung von 16-Oxa-bicyclo[11.2.1]hexadecan-2-on als Verbindung der Formel (Ia)

### 1.Stufe: Reduktion

In einen 250 ml Rührwerk wurden 22,2 g (0,1 mol) Cyclopentadec-4-en-1-on in 100 ml Methanol unter Rühren und Kühlen bei 20°C mit einer Lösung aus 5 g Wasser, 20 mg NaOH und 1,3 g Natriumboranat versetzt. Danach wurde 4 h bei 20°C nachgerührt. Zur Aufarbeitung wurde das Methanol am Rotationsverdampfer entfernt, der Rückstand mit 100 g Toluol und 100 g Wasser versetzt. Nach Trennung der Phasen wurde die organische Phase einmal mit Wasser gewaschen und das Toluol entfernt. Rohausbeute: 23,3 g.

GC-Auswertung (20m ZB-WAX, Innendurchmesser 0,18µm / 60-9-220°C Kaltaufgabesystem)

Das Rohprodukt wurde ohne weitere Reinigung in der 2. Stufe umgesetzt.

### 2. Stufe: Epoxidation

In einen 500 ml Rührwerk wurden 23,3 g Rohprodukt 1. Stufe in 250 ml Methylenchlorid, mit 25 g Natriumacetat vorgelegt. Anschließend wurde unter Rühren und Kühlen bei 18-25°C innerhalb von 2 Stunden 23,4 g (0,12 mol) Peressigsäure 38%ig zugetropft. Danach wurde 4 Stunden bei Raumtemperatur nachgerührt. Die Reaktionslösung wurde mit Wasser Eisen(II)sulfatlsg. Und Sodalsg. gewaschen und eingeengt. Rohausbeute: 24,9 g.

GC-Auswertung (20m ZB-WAX, Innendurchmesser 0,18µm / 60-9-220°C Kaltaufgabesystem)

Das Rohprodukt wurde ohne weitere Reinigung in der 3. Stufe umgesetzt.

### 3. Stufe: Cyclisierung

In einen 250 ml Rührwerk wurden 24,9 g Rohprodukt 2. Stufe in 200 ml Toluol unter Rühren bei 20°C mit 25 mg p-Toluolsulfonsäure versetzt. Danach wurde 4 h bei 20°C nachgerührt. Zur Aufarbeitung wurde das Reaktionsgemisch mit Natriumhydrogencarbonatlsg. gewaschen. Nach Trennung der Phasen wurde die organische Phase einmal mit Wasser gewaschen und das Toluol entfernt. Rohausbeute: 24,2 g.

GC-Auswertung (20m ZB-WAX, Innendurchmesser 0,18µm / 60-9-220°C Kaltaufgabesystem)

Das Rohprodukt wurde ohne weitere Reinigung in der 4. Stufe umgesetzt.

### 4. Stufe: Oxidation

In einen 2 I Rührwerk wurden 24,2 g Rohprodukt 3. Stufe in 1000 ml Methylenchlorid gelöst und unter Rühren bei 20-25°C mit 56,8 g (0,15 mol) Pyridiniumdichromat versetzt. Danach wurde 4 h bei 20°C nachgerührt. Zur Aufarbeitung wurde das Reaktionsgemisch über Kieselgel filtriert, mit verd. Schwefelsäure und Natriumhydrogencarbonatlsg. gewaschen und das Methylenchlorid entfernt. Rohausbeute: 23,8 g (ca. 1:1 E/Z-lsomere).

GC-Auswertung (20m ZB-WAX, Innendurchmesser 0,18µm / 60-9-220°C Kaltaufgabesystem)

Das Rohprodukt wurde über Säulenchromatographie gereinigt.

Es wurden die spektroskopischen Daten von hergestellten 16-Oxa-bicyclo[11.2.1]hexadecan-2-on ermittelt. Die Daten der E/Z Isomere sind nachfolgend angegeben.

### 16-Oxa-bicyclo[11.2.1]hexadecan-2-on Z-Isomer

¹H-NMR (CDCl₃, 400 MHZ, TMS= 0): 4,2(J=8, J=6,9, 1 H); 4,0(J=8,6, J=7,3, J=6,2, J=2,7, 1 H); 2,7(1 H); 2,5 (1 H); 2,1 (1 H); 1,50-1,78 (3H); 1,20-1,78(18H).

¹³C-NMR (CDCl₃, 100 MHz): 214,1 (s), 84,1 (d), 71,5(d), 37,8(t), 34,1 (t), 31,4(t), 28,4(t), 27,1 (t), 26,9(t), 25,8(t),. 25,6 (t), 25,3(t), 25,25(t), 24,4(t), 22,8(t).

MS: m/z (%) = 238 (20, M⁺), 210 (21), 166 (7), 126 (11), 111 (18), 97 (47), 84 (68), 71 (87), 55 (100), 41 (85), 29(34).

### Geruchsbeschreibung Z-Isomer: starker sauberer Moschusgeruch, interessante feine erogene Duftnote, sehr natürlicher Geruchseindruck.

### 16-Oxa-bicyclo[11.2.1]hexadecan-2-on E-Isomer

¹H-NMR (CDCl₃, 400 MHZ, TMS= 0): 4,35(J=8,2, J=6,8, 1 H); 3,8(J=4,65, 1 H); 2,7(J=5,4, J=12,7, J=10,9, 1H); 2,4(J=5,4, J=12,7, J=10,9, 1H); 2,2(1H); 2,05(1 H); 1,50-1,75(4H)1,20-1,50(16H).

¹³C-NMR (CDCl₃, 100 MHz): 215,8 (s), 83,0(d), 79,4(d), 37,5(t), 33,6(t), 32,7 (t), 27,7(t), 26,95(t), 26,35(t), 25,3(t), 25,1(t), 25,09(t), 24,6(t), 24,1(t), 22,6(t).

MS: m/z (%) = 238 (13, M⁺), 210 (23), 166 (7), 126 (10), 111 (16), 97 (47), 84 (74), 71 (89), 55 (100), 41 (85), 29(34).

### Geruchsbeschreibung Z-Isomer: starker sauberer Moschusgeruch, interessante feine erogene Duftnote, sehr natürlicher Geruchseindruck.

### Beispiel 4: Riech-und/oder Aromastoffkomposition (Parfümkomposition)

| | |
|---|---|
| Agrumex LC | 10.00 |
| Amarocit® 10%ig in DPG | 10.00 |
| Ambroxid kristallin | 10.00 |
| Basilikumöl | 10.00 |
| Calone 1951 10%ig in DPG | 10.00 |
| Cedernholzöl | 10.00 |
| Cedrol kristallin | 50.00 |
| Citral 10%ig in DPG | 10.00 |
| Citonellol | 5.00 |
| Cumarin | 10.00 |
| Cyclogalbanat® 10%ig in DPG | 15.00 |
| Dihydromyrcenol | 80.00 |
| Farenal® 10%ig in DPG | 5.00 |
| Galbex 10%ig in DPG | 25.00 |
| Globalide® | 80.00 |
| Globanone® | 40.00 |
| Hedion | 90.00 |
| Helional | 20.00 |
| Heliotropin | 5.00 |
| Hexenol cis-3 10%ig in DPG | 15.00 |
| Hexenylsalicylat cis-3 | 10.00 |
| Beta-Ionon | 5.00 |
| Iso E Super | 180.00 |
| Isodamascon® 10%ig in DPG | 10.00 |
| Isomuscone (Cyclohexadecanon) | 20.00 |
| Isoraldein 70 | 20.00 |
| Ketamber 10%ig in TEC | 25.00 |
| Lavandinöl Grosso nat. | 15.00 |
| Lilial | 20.00 |
| Linalool | 20.00 |
| Linalylacetat | 40.00 |
| Mandarinenöl brasil. grün | 50.00 |
| Timberol® | 40.00 |
| Vanillin | 5.00 |
| Veloutone 10%ig in DPG | 20.00 |
| Ysamber K® | 10.00 |
| Gesamt | 1000.00 |

### DPG: Dipropylenglycol, TEC = Triethylcitrat

Geruchsbeschreibung der Parfümkomposition ohne Zusatz von Verbindung der Formel (I): holzig, blumig, herb

Nach Meinung der Parfümeure wird diese Parfümkomposition durch den Zusatz von 3 Gew.-% von Verbindung der Formel (I) aus Beispiel 1 frischer, strahlender, abgerundeter und harmonischer, wobei eine Moschusnote hinzukommt und die holzigen und blumigen Aspekte verstärkt werden. Die eingesetzte Verbindung der Formel I verleiht der Komposition durch ihren Eigengeruch sowie durch ihre modifizierende und verstärkende Wirkung (Booster-Wirkung) einen eigenen Charakter und verbinden die unterschiedlichen geruchlichen Elemente.

### Beispiel 5: Riech-und/oder Aromastoffkomposition (Parfümkomposition)

| | |
|---|---|
| Allylcyclohexylpropionat | 3.00 |
| Amylsalicylat | 2.00 |
| Benzylacetat | 64.00 |
| Citronellol | 122.00 |
| Citral 10% ig in DPG | 2.00 |
| Cyclamenaldehyd | 10.00 |
| Dihydromyrcenol | 3.00 |
| Dimethylbenzylcarbinylacetat | 3.00 |
| Ethylsalicylat 10% ig in DPG | 2.00 |
| Eugenol | 3.00 |
| Indoflor 10%ig in DPG | 16.00 |
| Galaxolide 50%ig in DPG | 164.00 |
| Geraniol | 35.00 |
| Dihydromethyljasmonate | 6.00 |
| Heliotropin | 4.00 |
| Hexylzimtaldehyd | 121.00 |
| Vertocitral | 4.00 |
| Hedion | 42.00 |
| Indol | 2.00 |
| Isobutylsalicylat | 6.00 |
| Lavandinöl Grosso nat. | 6.00 |
| Acetylcedren | 10.00 |
| Majantol | 190.00 |
| Linalool | 35.00 |
| Linalylacetat | 10.00 |
| Methylanthranilat 10%ig in DPG | 5.00 |
| Nerol | 10.00 |
| Orangenöl | 6.00 |
| Phantolide | 4.00 |
| Phenylacetaldehyddimethylacetal | 6.00 |
| Phenylethylalkohol | 75.00 |
| Florol | 6.00 |
| Sandelholzöl | 3.00 |
| Sandranol | 16.00 |
| Trifernal | 2.00 |
| Tonalid | 2.00 |
| Gesamt | 1000.00 |

### DPG: Dipropylenglycol

Geruchsbeschreibung der Parfümkomposition ohne Zusatz von Verbindungen der Formel (Ia): blumig, Maiglöckchen.

Nach Meinung der Parfümeure erwacht diese Parfümkomposition durch den Zusatz von 1 Gew.-% von Verbindung der Formel (Ia) aus Beispiel 3 zu neuem Leben. Der Eindruck von Blumigkeit wird erheblich verstärkt. Die Komposition wirkt strahlender, abgerundeter und harmonischer, wobei eine weiche natürliche moschus Note hinzukommt. Die eingesetzte Verbindung der Formel I verleiht der Komposition durch ihren Eigengeruch sowie durch ihre modifizierende und verstärkende Wirkung (Booster-Wirkung) einen eigenen Charakter und verbindet die unterschiedlichen geruchlichen Elemente.

### Beispiel 6: Shampoo

Die Verbindungen der Formel (I) und/oder Formel (Ia) aus Beispiel 1 u. 3 wurden jeweils separat in einer Dosierung von 0,5 Gew.-% in eine Shampoo-Grundmasse folgender Zusammensetzung eingearbeitet:

| | |
|---|---|
| Natriumlaurylethersulfat (z.B. Texapon NSO, Fa. Cognis Deutschland GmbH) | 12% |
| Cocamidopropylbetain (z.B. Dehyton K, Fa. Cognis Deutschland GmbH) | 2% |
| Natriumchlorid | 1,4% |
| Citronensäure | 1,3% |
| Phenoxyethanol, Methyl-, Ethyl-, Butyl-, und Propylparaben | 0,5% |
| Wasser | 82,8% |

Der pH-Wert der Shampoo-Grundmasse lag bei etwa 6. Hieraus wurden 100 mL einer 20 Gew.%-igen wässrigen Shampoo-Lösung, die entweder die Verbindung aus Beispiel 1 oder Beispiel 3 umfasst, hergestellt. In diesen Shampoo-Lösungen wurden 2 Haarsträhnchen gemeinsam für 2 Minuten gewaschen und anschließend 20 Sekunden unter fließendem handwarmen Wasser gespült. Es wurde für jede Shampoo Lösung jeweils eine Haarsträhne nass in Aluminiumfolie eingepackt und die zweite Haarsträhne mit einem Fön getrocknet. Beide Haarsträhnen wurden von einem Panel geruchlich beurteilt.

Geruchsbeschreibung jeweils: starker sauberer Moschusgeruch, interessante feine erogene Duftnote sehr natürlicher Geruchseindruck.

### Beispiel 7: Weichspüler

Die Parfümkomposition aus Beispiel 4 (nach Zusatz von 3 Gew.-% von Verbindung Formel (I) aus Beispiel 1 wurde in einer Dosierung von 0,5 Gew.-% in eine Weichspüler-Grundmasse folgender Zusammensetzung eingearbeitet

| | |
|---|---|
| Quarternäres Ammoniummethosulfat (Esterquat), ca. 90% | 5,5% |
| (z.B. Rewoquat WE 18, Fa. Witco Surfactants GmbH) | |
| Alkyldimethylbenzylammoniumchlorid, ca. 50% | 0,2% |
| (z.B. Preventol R50, Fa. Bayer AG) | |
| Farblösung, ca. 1%-ig | 0,3% |
| Wasser | 94,0% |

Der pH-Wert der Weichspüler-Grundmasse lag im Bereich von 2 bis 3. Zwei Stofflappen wurden mit 370 g einer 1%-igen wässrigen Weichspüler-Lösung auf Basis der 0,5% Gew.-% von Verbindung Formel (I) umfassenden Weichspüler-Grundmasse in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung jeweils: starker sauberer Moschusgeruch, interessante feine erogene Duftnote sehr natürlicher Geruchseindruck.

### Beispiel 8: Waschpulver

Die Parfümölkomposition aus Beispiel 4 (nach Zusatz von 6 Gew.-% von Verbindung Formel (Ia) aus Beispiel 3) wurde in einer Dosierung von 0,4 Gew.-% in eine Waschpulver-Grundmasse der folgenden Rezeptur eingearbeitet

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer | |
| DOW CORNING(R) 2-4248S | |
| POWDERED ANTIFOAM, | |
| Silikonöl auf Zeolith als Trägermaterial | 3,9 % |
| Zeolith 4A | 28,3 % |
| Na-Carbonat | 11,6 % |
| Na-Salz eines Copolymers aus Acryl- | |
| und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis | |
| (methylen)]]tetrakis-phosphonsäure, Natriumsalz) | |
| Optischer Aufheller | 0,2 % |
| Na-Sulfat | 6,5 % |
| Protease | 0,4 % |
| Natriumperborattetrahydrat | 22,0 % |
| TAED | 1,0 % |

Zwei Stofflappen wurden mit 370 g einer 1%-igen wässrigen Waschpulverlauge auf Basis des 0,4 Gew.-% der Parfümölkomposition aus Beispiel 4 umfassenden Waschpulver-Grundmasse (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt, und einer zum Trocknen aufgehängt. Anschlie-ßend wurden beide Lappen durch ein Panel geruchlich beurteilt.

Geruchsbeschreibung jeweils: starker sauberer Moschusgeruch, interessante feine erogene Duftnote sehr natürlicher Geruchseindruck.

### Beispiel 9 : Riech-und/oder Aromastoffkomposition (Parfümkomposition)

| | |
|---|---|
| Allylcyclohexylpropionat | 3.00 |
| Amylsalicylat | 2.00 |
| Benzylacetat | 64.00 |
| Citronellol | 122.00 |
| Citral 10% ig in DPG | 2.00 |
| Cyclamenaldehyd | 10.00 |
| Dihydromyrcenol | 3.00 |
| Dimethylbenzylcarbinylacetat | 3.00 |
| Ethylsalicylat 10% ig in DPG | 2.00 |
| Eugenol | 3.00 |
| Indoflor 10%ig in DPG | 16.00 |
| Galaxolide 50%ig in DPG | 164.00 |
| Geraniol | 35.00 |
| Dihydromethyljasmonate | 6.00 |
| Heliotropin | 4.00 |
| Hexylzimtaldehyd | 121.00 |
| Vertocitral | 4.00 |
| Hedion | 42.00 |
| Indol | 2.00 |
| Isobutylsalicylat | 6.00 |
| Lavandinöl Grosso nat. | 6.00 |
| Acetylcedren | 10.00 |
| Majantol | 190.00 |
| Linalool | 35.00 |
| Linalylacetat | 10.00 |
| Methylanthranilat 10%ig in DPG | 5.00 |
| Nerol | 10.00 |
| Orangenöl | 6.00 |
| Phantolide | 4.00 |
| Phenylacetaldehyddimethylacetal | 6.00 |
| Phenylethylalkohol | 75.00 |
| Florol | 6.00 |
| Sandelholzöl | 3.00 |
| Sandranol | 16.00 |
| Trifernal | 2.00 |
| Tonalid | 2.00 |
| Gesamt | 1000.00 |

### DPG: Dipropylenglycol

Geruchsbeschreibung der Parfümkomposition ohne Zusatz von Verbindungen der Formel (I) und Formel (Ia): blumig, Maiglöckchen.

Nach Meinung der Parfümeure erweckt diese Parfümkomposition durch den Zusatz von 2 Gew.-% einer 1:1 Mischung der Verbindungen Formel (I) und (Ia) aus Beispiel 1 und 3 den Eindruck von weicher natürlicher Blumigkeit. Die Komposition wirkt strahlender, abgerundeter und harmonischer, wobei eine angenehme erogene moschus Note hinzukommt. Die eingesetzte Verbindungen der Formel (I) und (Ia) verleihen der Komposition durch ihren Eigengeruch sowie durch ihre modifizierende und verstärkende Wirkung (Booster-Wirkung) einen eigenen Charakter und verbindet die unterschiedlichen geruchlichen Elemente.

## Patentansprüche

1. Verbindungen der Formel (I) und Formel (Ia): 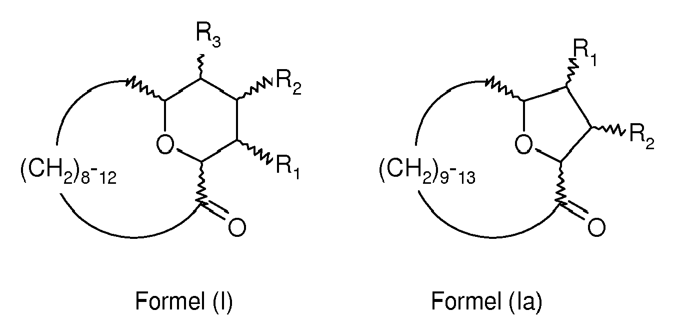 wobei R₁, R₂ und R₃ unabhängig voneinander Wasserstoff oder Methyl bedeuten.

2. Verbindungen gemäß Anspruch 1, wobei für Verbindungen der Formel (I)
R₁=Methyl sowie R₂ und R₃=Wasserstoff bedeuten oder
R₁ und R₃=Wasserstoff sowie R₂=Methyl bedeuten oder
R₁ und R₂=Wasserstoff sowie R₃=Methyl bedeuten oder
R₁, R₂ und R₃=Wasserstoff bedeuten.

3. Verbindungen gemäß Anspruch 1 oder 2 ausgewählt aus der Gruppe bestehend aus Formel (I):
14-Oxa-bicyclo[8.3.1]tetradecan-2-on, 15-Oxa-bicyclo[9.3.1]pentadecan-2-on, 16-Oxa-bicyclo[10.3.1]hexadecan-2-on, 17-Oxa-bicyclo[11.3.1]heptadecan-2-on, 18-Oxa-bicyclo[12.3.1]octadecan-2-on, 13-Methyl-16-Oxa-bicyclo[10.3.1]hexadecan-2-on, 14-Methyl-16-Oxa-bicyclo[10.3.1]hexadecan-2-on und 15-Methyl-16-Oxa-bicyclo[10.3.1]hexadecan-2-on.

4. Verbindungen gemäß Anspruch 1, wobei für Verbindungen der Formel (Ia)
R₁=Methyl sowie R₂ =Wasserstoff bedeuten oder
R₁ =Wasserstoff sowie R₂=Methyl bedeuten oder
R₁ sowie R₂=Methyl bedeuten oder
R₁ und R₂ =Wasserstoff bedeuten.

5. Verbindungen gemäß Anspruch 1 oder 4 ausgewählt aus der Gruppe bestehend aus Formel (Ia):
15-Oxa-bicyclo[9.2.1]tetradecan-2-on, 15-Oxa-bicyclo[10.2.1]pentadecan-2-on, 16-Oxa-bicyclo[11.2.1]hexadecan-2-on, 17-Oxa-bicyclo[12.2.1]heptadecan-2-on, 18-Oxa-bicyclo[13.2.1]octadecan-2-on, 13-Methyl-16-Oxa-bicyclo[9.3.1]hexadecan-2-on, 14-Methyl-16-Oxa-bicyclo-[11.2.1]hexadecan-2-on und 15-Methyl-16-Oxa-bicyclo [11.2.1]hexadecan-2-on.

6. Riech- und/oder Aromastoffkomposition umfassend oder bestehend aus
a) einer, zwei, drei, vier oder mehreren Verbindungen der Formel (I) und/oder Formel (Ia) gemäß einem der Ansprüche 1 bis 5
und
b) einem, zwei, drei oder mehreren weiteren Riech- und/oder Aromastoffen, die nicht Verbindungen der Formel (I) und Formel (Ia) darstellen.

7. Komposition gemäß Anspruch 6, **dadurch gekennzeichnet, dass** die Komposition eine Gesamtmenge der Verbindungen der Formel (I) und/oder Formel (Ia) in Komponente (a) umfasst, die ausreicht in der Komposition einen Geruch und/oder Geschmack mit einer, mehreren oder sämtlichen moschusartigen Noten zu vermitteln, zu modifizieren und/oder zu verstärken.

8. Komposition gemäß Anspruch 6 oder 7, **dadurch gekennzeichnet, dass** die Komposition eine Gesamtmenge einer, zwei, drei, vier oder mehrerer Verbindungen der Formel (I) und/oder Formel (Ia) in Komponente (a) umfasst, die ausreicht
dieser Komposition eine (Aus)Strahlung , Abrundung und/oder Harmonie zu verleihen
und/oder
die Geruchs- und/oder Geschmacksnoten des weiteren Riech- und/oder Aromastoffes oder eines, zwei, drei oder mehrerer der weiteren Riech-und/oder Aromastoffe in Komponente (b) zu verstärken.

9. Komposition gemäß einem der Ansprüche 6 bis 8, **dadurch gekennzeichnet, dass**
ein, zwei, drei oder mehrere Riech- und/oder Aromastoffe der Komponente (b) eine holzige Geruchsnote und/oder Geschmacksnote oder
ein, zwei, drei oder mehrere Riech- und/oder Aromastoffe der Komponente (b) eine blumige Geruchsnote und/oder Geschmacksnote oder
ein, zwei, drei oder mehrere Riech- und/oder Aromastoffe der Komponente (b) eine holzige und blumige Geruchsnote und/oder Geschmacksnote aufweisen.

10. Komposition gemäß einem der Ansprüche 6 bis 9, **dadurch gekennzeichnet, dass** die Komposition zusätzlich ein oder mehrere Lösungsmittel umfasst.

11. Komposition gemäß einem der Ansprüche 6 bis 10, **dadurch gekennzeichnet, dass** die Komposition teilweise oder vollständig an einen oder mehrere Trägerstoffe adsorbiert ist.

12. Komposition gemäß einem der Ansprüche 6 bis 11, **dadurch gekennzeichnet, dass** die Komposition teilweise oder vollständig mikroverkapselt und/oder sprühgetrocknet oder als Einschluss-Komplexe oder als Extrusions-Produkte vorliegt.

13. Komposition gemäß Anspruch 12, **dadurch gekennzeichnet, dass** die Komposition mit geeigneten Materialien zur gezielten Riech- und/oder Aromastofffreisetzung beschichtet ist.

14. Parfümierte Kosmetik- oder Haushalts-Produkte umfassend oder bestehend aus
a) ein, zwei, drei, vier oder mehreren Verbindungen der Formel (I) und/oder Formel (Ia) gemäß einem der Ansprüche 1 bis 5 und/oder
b) einer, zwei, drei oder mehreren Riech- und/oder Aromastoffkompositionen gemäß einem der Ansprüche 6 bis 13
und
c) ein oder mehreren weiteren Grund-, Hilfs- und/oder Zusatzstoffen.

15. Produkte gemäß Anspruch 14, **dadurch gekennzeichnet, dass** das Produkt ausgewählt wird aus der Gruppe bestehend aus:
Parfüm-Extraits, Eau de Parfums, Eau de Toilettes, Rasierwässer, Eau de Colognes, Pre-shave-Produkte, Splash-Colognes,
parfümierte Erfrischungstücher,
saure, alkalische und neutrale Reinigungsmittel,
Desinfektionsmittel, Oberflächendesinfektionsmittel,
Luftverbesserer in flüssiger, gelartiger oder auf einem festen Träger aufgebrachter Form, Aerosolsprays,
Wachse und Polituren,
Körperpflegemittel,
Haarpflegeprodukten,
Deodorantien und Antiperspirantien,
Produkte der dekorativen Kosmetik,
Kerzen, Lampenöle, Räucherstäbchen, Insektizide, Repellentien und Treibstoffe.

16. Aromatisierte Produkte, die der Ernährung, der Mundpflege und/oder dem Genuss dienen sowie Halbfertigware hierzu oder Würzmischungen umfassend oder bestehend aus
a) ein, zwei, drei, vier oder mehreren Verbindungen der Formel (I) und/oder Formel (Ia) gemäß einem der Ansprüche 1 bis 5 und/oder
b) einer, zwei, drei oder mehreren Riech- und/oder Aromastoffkompositionen gemäß einem der Ansprüche 6 bis 13
und
c) ein oder mehreren weiteren Grund-, Hilfs- und/oder Zusatzstoffen.

17. Produkte gemäß Anspruch 16, **dadurch gekennzeichnet, dass** das Produkt, dass der Ernährung und/oder dem Genuss dient, ein Halbfertigprodukt oder eine Würzmischung ausgewählt wird aus der Gruppe bestehend aus:
Backwaren,
Süßwaren,
alkoholische oder nicht-alkoholische Getränke,
Instantgetränke,
Fleischprodukte,
Eier oder Eiprodukte,
Getreideprodukte,
Milchprodukte,
Produkte aus Sojaprotein oder anderen Sojabohnen-Fraktionen,
Fruchtzubereitungen,
Gemüsezubereitungen,
Knabberartikel,
Produkte auf Fett- und Ölbasis oder Emulsionen derselben,
sonstige Fertiggerichte und Suppen.

18. Verwendung einer, zwei, drei, vier oder mehrerer Verbindungen der Formel (I) und/oder Formel (Ia) gemäß einem der Ansprüche 1 bis 5 als Riech-und/oder Aromastoffe.

19. Verwendung gemäß Anspruch 18 als Riech- und/oder Aromastoffe mit moschusartigen Noten.

20. Verwendung gemäß Anspruch 18 oder 19, wobei ein, zwei, drei, vier oder mehrere Verbindungen der Formel (I) und/oder Formel (Ia) gemäß einem der Ansprüche 1 bis 5 in Riech- und/oder Aromakompositionen gemäß einem der Ansprüche 6 bis 13, einem parfümierten Produkt gemäß Anspruch 14 oder 15 oder einem aromatisierten Produkt gemäß Anspruch 16 oder 17 als Riech- und/oder Aromastoff verwendet wird.

21. Verwendung nach einem der Ansprüche 18 bis 20, wobei ein, zwei, drei, vier oder mehrere Verbindungen der Formel (I) und/oder Formel (Ia) zum Vermitteln, Modifizieren und/oder Verstärken eines Geruchs oder Geschmacks mit einer, mehreren oder sämtlichen moschusartigen Noten verwendet werden.

22. Verwendung nach einem der Ansprüche 18 bis 21, wobei eine oder mehrere Verbindungen der Formel (I) und/oder Formel (Ia) als Booster verwendet werden.

23. Verwendung nach einem der Ansprüche 18 bis 22, wobei eine, zwei, drei, vier oder mehrere Verbindungen der Formel (I) und/oder Formel (Ia) zur Erhöhung der Substantivität weiterer Riech- und/oder Aromastoffe auf Haut, Haare und/oder Textilfasern verwendet werden.

24. Verwendung nach einem der Ansprüche 18 bis 23, wobei eine, zwei, drei, vier oder mehrere Verbindungen der Formel (I) und/oder Formel (Ia) zur Erhöhung der Retention weiterer Riech- und/oder Aromastoffe verwendet werden.

25. Verwendung nach einem der Ansprüche 18 bis 24, wobei eine, zwei, drei, vier oder mehrere Verbindungen der Formel (I) und/oder Formel (Ia) verwendet werden, um (Aus)Strahlung, Abrundung und/oder Harmonie zu verleihen und/oder Geruchs- und/oder Geschmacksnoten in Riech- und/oder Aromastoffkomposition zu verstärken.

26. Verfahren zum Vermitteln, Modifizieren und/oder Verstärken eines Geruchs oder Geschmacks mit einer, mehreren oder sämtlichen moschusartigen Noten, wobei eine sensorisch wirksame Menge einer, zwei, drei, vier oder mehrerer Verbindungen der Formel (I) und/oder Formel (Ia) gemäß einem der Ansprüche 1 bis 5
oder eine sensorisch wirksame Menge einer oder mehrere eine, zwei, drei, vier oder mehrere Verbindungen der Formel (I) und/oder Formel (Ia) gemäß einem der Ansprüche 1 bis 5 umfassende Riechstoff- und/oder Aromastoffkompositionen gemäß einem der Ansprüche 6 bis 13 mit einem Erzeugnis in Kontakt gebracht oder gemischt werden.

27. Verfahren zur Herstellung einer oder mehrerer Verbindungen der Formel (I) gemäß einem der Ansprüche 1 bis 3
umfassend oder bestehend aus folgenden Schritten:
a) Epoxidation einer Verbindung der Formel (II) zu einer entsprechenden Verbindung der Formel (III) 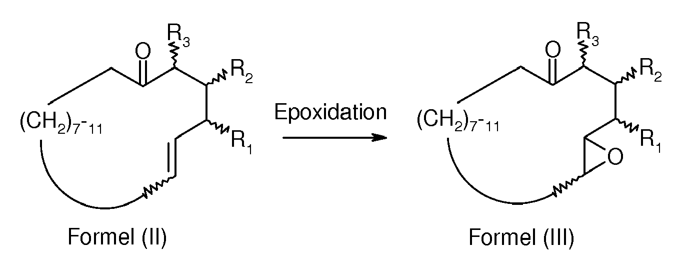 wobei R₁, R₂ und R₃ die oben dargestellten Bedeutungen haben,
b) anschließende Reduktion der entsprechenden Verbindung der Formel (III) zu einer entsprechenden Verbindung der Formel (IV) 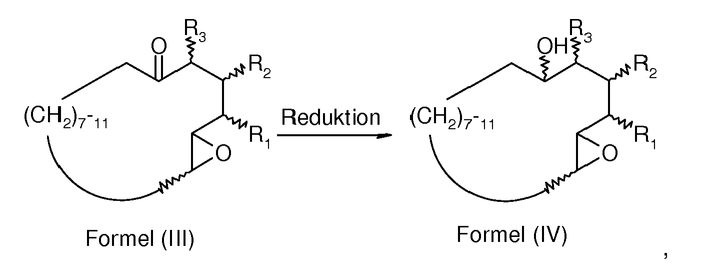 wobei R₁, R₂ und R₃ die oben dargestellten Bedeutungen haben,
c) anschließende Cyclisierung der entsprechenden Verbindung der Formel (IV) zu einer entsprechenden Verbindung der Formel (V) 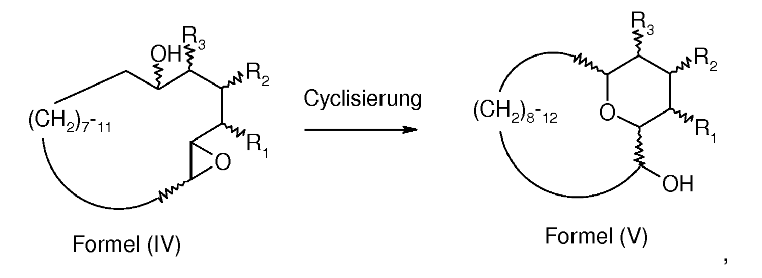 wobei R₁, R₂ und R₃ die oben dargestellten Bedeutungen haben und
d) anschließende Oxidation der entsprechenden Verbindung der Formel (V) zu einer entsprechenden Verbindung der Formel (I) 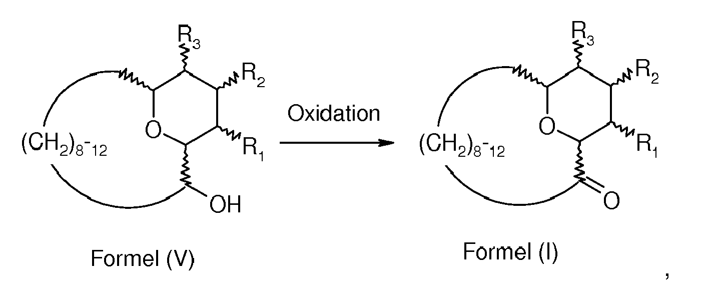
wobei R₁, R₂ und R₃ die oben dargestellten Bedeutungen haben.

28. Verfahren zur Herstellung einer oder mehrerer Verbindungen der Formel (Ia) gemäß einem der Ansprüche 1, 4 und 5
umfassend oder bestehend aus folgenden Schritten:
a) Reduktion einer Verbindung der Formel (IIa) zu einer entsprechenden Verbindung der Formel (IIIa) 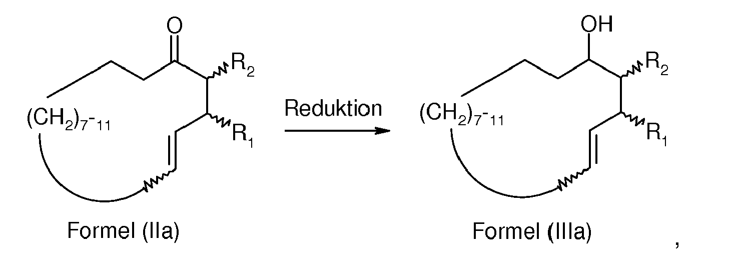 wobei R₁ und R₂ die oben dargestellten Bedeutungen haben,
b) anschließende Epoxidation der entsprechenden Verbindung der Formel (IIIa) zu einer entsprechenden Verbindung der Formel (IVa) 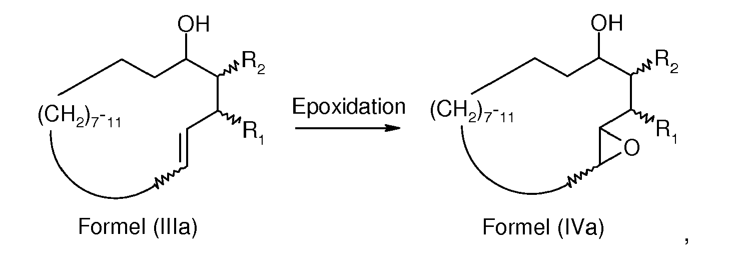 wobei R₁ und R₂ die oben dargestellten Bedeutungen haben,
c) anschließende Cyclisierung der entsprechenden Verbindung der Formel (IVa) zu einer entsprechenden Verbindung der Formel (Va) 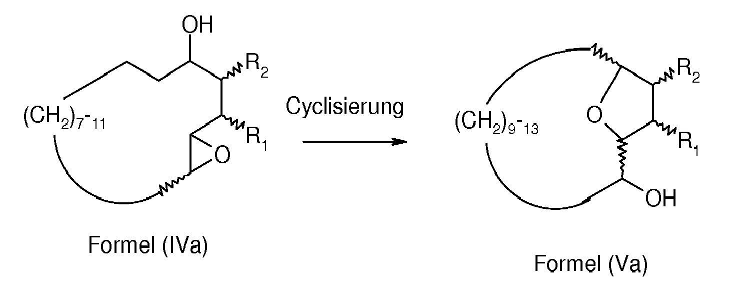 wobei R₁ und R₂ die oben dargestellten Bedeutungen haben und
d) anschließende Oxidation der entsprechenden Verbindung der Formel (Va) zu einer entsprechenden Verbindung der Formel (Ia) 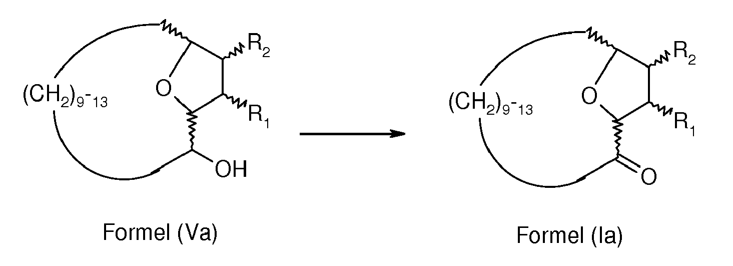
wobei R₁ und R₂ die oben dargestellten Bedeutungen haben.
